(19) Europäisches Patentamt / European Patent Office / Office européen des brevets

(11) EP 4 553 142 A1

# (12) EUROPEAN PATENT APPLICATION

published in accordance with Art. 153(4) EPC

(43) Date of publication:
**14.05.2025 Bulletin 2025/20**

(21) Application number: **23835172.0**

(22) Date of filing: **24.05.2023**

(51) International Patent Classification (IPC):
***C12M 1/00*** *(2006.01)* ***B32B 3/12*** *(2006.01)*
***C12N 5/071*** *(2010.01)*

(52) Cooperative Patent Classification (CPC):
**B32B 3/12; C12M 1/00; C12N 5/06**

(86) International application number:
**PCT/JP2023/019310**

(87) International publication number:
**WO 2024/009636 (11.01.2024 Gazette 2024/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.07.2022 JP 2022109780**

(71) Applicant: **Mitsui Chemicals, Inc.**
**Tokyo 104-0028 (JP)**

(72) Inventors:
- **ESASHIKA Katsuhiro**
  **Sodegaura-shi, Chiba 299-0265 (JP)**
- **SAKAJIRI Aya**
  **Sodegaura-shi, Chiba 299-0265 (JP)**
- **MATSUGI Tomoaki**
  **Tokyo 104-0028 (JP)**

(74) Representative: **J A Kemp LLP**
**80 Turnmill Street**
**London EC1M 5QU (GB)**

## (54) WELL PLATE AND CULTURE METHOD

(57) Provided are a well plate and a culture method which are excellent in ability to attach cells, etc., and exhibit a low drug adsorption property.

The well plate including a bottom surface component and a well lateral wall component, wherein the bottom surface component and the well lateral wall component are bonded to each other to form at least one well, the bottom surface component and the well lateral wall component are formed of different materials, and a surface free energy of the well lateral wall component is 18.0 to 34.0 mN/m.

EP 4 553 142 A1

## Description

### Technical Field

An aspect of the present invention relates to a well plate and a culture method.

### Background Art

In culture of cells, tissues and organs (hereinafter, also referred to collectively as " cells, etc.") in medical or drug development business including, for example, drug screening, well plates provided one or more wells including a bottom surface and a lateral wall are widely used. The well plate is generally formed so that a bottom surface and a lateral wall are integrated. As a material used for well plates, polystyrene is commonly used from the viewpoint of transparency and cost.

When culturing cells, etc. using a well plate, a culture medium and cells, etc. are placed in the well, and cells, etc. are cultured on the well bottom surface. When placing the culture medium in the well, the culture medium is typically placed into the well by running along the well lateral wall so as not to damage cells, etc. on the well bottom surface.

However, it is known that polystyrene is likely to absorb a drug and a protein, and has a high drug adsorption property. Therefore, use of an integrally formed well plate made of polystyrene had a problem that when a culture medium is run along a well lateral wall, a drug and a protein in the culture medium are absorbed by the polystyrene forming the well lateral wall, so that the drug and the protein are not sufficiently supplied to cells, etc. in the well.

As a plastic molded article to which DNA and proteins do not easily attach, Patent Literature 1 discloses a plastic molded article used in the biotechnology field, which is made of a molded article formed using a plastic material obtained by providing thermoplastic as a base material, and adding a siloxane copolymer resin to the thermoplastic, followed by mixing.

### Citation List

### Patent Literature

[Patent Literature 1] JP2022-064159A

### Summary of Invention

### Technical Problem

The plastic molded article described in Patent Literature 1 has improved water-repellent and oil-repellent effects on a plastic surface, and can exhibit functions equivalent to those when a silicone material is applied. Studies by the inventors of the present invention have revealed that the plastic molded article described in Patent Literature 1 has a drug adsorption property lower than that of a polystyrene well plate, and is excellent in drug adsorption property, but has high water-repellent and oil-repellent effects, and is likely to repel not only a drug but also, for example, cells, etc., a culture medium and a coating agent. That is, the plastic molded article described in Patent Literature 1 had room for improvement in ability to attach cells, etc.

Accordingly, an aspect of the present invention provides a well plate and a culture method which are excellent in ability to attach cells, etc., and exhibit a low drug adsorption property.

### Solution to Problem

The inventors of the present invention have conducted diligent research in order to solve the problems described above. As a result, the present inventors have found that the problems described above can be solved by having the following configuration, leading to completion of the present invention.

The aspect of the present invention relates to, for example, the following [1] to [14].

[1] A well plate comprising a bottom surface component and a well lateral wall component, wherein the bottom surface component and the well lateral wall component are bonded to each other to form at least one well, the bottom surface component and the well lateral wall component are formed of different materials, and a surface free energy of the well lateral wall component is 18.0 to 34.0 mN/m.
[2] The well plate according to [1], wherein the well lateral wall component comprises a thermoplastic resin.
[3] The well plate according to [2], wherein the thermoplastic resin is a polyolefin-based resin.
[4] The well plate according to [3], wherein the polyolefin-based resin is at least one selected from a cyclic olefin-based

copolymer (α), a 4-methyl-1-pentene polymer (β), a propylene-based polymer (γ) and a cyclic olefin-based polymer (δ) (except for the cyclic olefin-based copolymer (α)).
[5] The well plate according to [4], wherein the well lateral wall component further comprises a silylated polyolefin (ε).
[6] The well plate according to any one of [1] to [5], wherein a glass transition temperature of the bottom surface component is -40°C or higher as measured in accordance with JIS-K 7121: 1987.
[7] The well plate according to any one of [1] to [6], wherein a total light transmittance of the bottom surface component is 80.0% or more as measured in accordance with JIS-K-7361-1.
[8] The well plate according to any one of [1] to [7], wherein a thickness of the bottom surface component is 20 to 500 μm.
[9] The well plate according to any one of [1] to [8], wherein a culture surface of the bottom surface component is coated with at least one coating agent selected from a natural polymeric material, a synthetic polymeric material and an inorganic material.
[10] The well plate according to any one of [1] to [9], wherein a water contact angle of the culture surface of the bottom surface component is 50 to 100°.
[11] The well plate according to any one of [1] to [10], wherein a glass transition temperature of the well lateral wall component is 30°C or higher as measured by solid viscoelasticity temperature dispersion measurement.
[12] The well plate according to any one of [1] to [11], wherein the glass transition temperature of the well lateral wall component is 120°C or higher as measured by solid viscoelasticity temperature dispersion measurement.
[13] The well plate according to any one of [1] to [12], wherein the surface free energy of the well lateral wall component is 18.0 to 24.3 mN/m.
[14] A method for culturing a cell, tissue or organ, comprising the step of culturing a cell, tissue or organ using the well plate according to any one of [1] to [13].

Advantageous Effects of Invention

According to an aspect of the present invention, it is possible to provide a well plate and a culture method which are excellent in ability to attach cells, etc., and exhibit a low drug adsorption property.

Description of Embodiments

The present invention will be specifically described below.
In the present disclosure, the notations of "XX or more and YY or less" and "XX to YY" representing a numerical range each mean a numerical range including the lower limit and the upper limit which are end points unless otherwise specified.
When numerical ranges are described in stages, the upper limit and the lower limit of each numerical range can be arbitrarily combined.

<Well plate>

A well plate according to an aspect of the present invention comprises a bottom surface component and a well lateral wall component, the bottom surface component and the well lateral wall component are bonded to each other to form at least one well, the bottom surface component and the well lateral wall component are formed of different materials, and a surface free energy of the well lateral wall component is 18.0 to 34.0 mN/m. Hereinafter, the well plate is also referred to as a well plate (X).
The well lateral wall component and the bottom surface component of the well plate (X) are bonded to each other so that cells, etc. or a culture medium in the well does not leak. A bonding may be any of mechanical bonding, material bonding or adhesive bonding.
The well plate (X) is a plate having at least one well. The well plate (X) is preferably a multi-well plate having a plurality of wells such as 6 wells, 12 wells, 24 wells, 48 wells, 96 wells, 384 wells and 1536 wells.
The well plate (X) may have a lid covering the upper part of the plate.
The well plate (X) can be used for a culture method comprising the step of culturing a cell, tissue or organ. The cell, tissue and organ are not particularly limited, and the well plate (X) can be used for culturing a known cell, tissue and organ.
Since the well plate (X) has excellent in ability to attach cells, etc., it is preferable that cells, etc. be adhesive. Cells, etc. are preferably cells, and more preferably adhesive cells because they are suitable for culturing on a culture surface of the well plate (X).
The method for producing the well plate (X) is not limited, and equipment used for the production is not also limited. The well plate (X) can be obtained by molding a well lateral wall component into a desired shape by a method such as extrusion molding, solution casting molding, injection molding or blow molding, and then bonding a bottom surface

component. The bonding method is not particularly limited, and examples thereof include mechanical bonding such as holding with a resilient spring component or locking with a screw component; material bonding such as fusion welding using, for example, heat, an ultrasonic wave, a laser or a high frequency dielectric phenomenon; and adhesive bonding with an adhesive or a pressure sensitive adhesive.

<Bottom surface component>

The bottom surface component constitutes the bottom surface of the well plate (X), and is bonded to the well lateral wall component to form at least one well. When bonded to the well lateral wall component to form at least one well, the bottom surface component constitutes the bottom surface of the well.

The material constituting the bottom surface component is not particularly limited as long as it is a material that differs from a material constituting the well lateral wall component. A known material (for example, a resin such as a thermosetting resin or a thermoplastic resin, or glass) can be used. Specifically, the bottom surface component can be formed of a similar material as that used to constitute the well lateral wall component, which is described later. However, the material is required to differ from a material constituting the well lateral wall component. The term "different materials" means that main materials constituting the bottom surface component and the well lateral wall component (the material that is contained in the largest amount by mass among the materials constituting the component) are different. When the main material is a (co)polymer, the term "different materials" means exclusion of a case where the types and proportions of the constituent units constituting the polymers are the same. For example, even if the material constituting the bottom surface component and the material constituting the well lateral wall component contain the common constituent unit, the material constituting the bottom surface component and the material constituting the well lateral wall component are different when the ratio of the constituent unit differs between the material constituting the bottom surface component and the material constituting the well lateral wall.

The material constituting the bottom surface component preferably contains a thermoplastic resin or glass, and more preferably contains a polyolefin-based resin. The polyolefin-based resin is preferably at least one selected from a cyclic olefin-based copolymer ($\alpha$), a 4-methyl-1-pentene polymer ($\beta$), a propylene-based polymer ($\gamma$) and a cyclic olefin-based polymer ($\delta$) (except for the cyclic olefin-based copolymer ($\alpha$)), more preferably a 4-methyl-1-pentene polymer ($\beta$) or a propylene-based polymer ($\gamma$), and further more preferably a 4-methyl-1-pentene polymer ($\beta$).

The bottom surface component preferably has a glass transition temperature (Tg) measured in accordance with JIS-K 7121: 1987 of -40°C or higher, and more preferably -20°C or higher. The upper limit of Tg is not particularly limited, and may be, for example, 600°C or lower.

When the Tg of the bottom surface component is within the above-described range, excellent flexibility and strength and an excellent low drug adsorption property are achieved.

The bottom surface component preferably has a total light transmittance measured in accordance with JIS-K-7361-1 of 80.0% or more, and more preferably 90.0% or more. The upper limit of the total light transmittance is not particularly limited, and may be, for example, 100.0% or less, or less than 100.0%.

When the total light transmittance of the bottom surface component is within the above-described range, the bottom surface component has excellent transparency, so that cells, etc. are easily observed both with the naked eye and under a microscope.

The thickness of the bottom surface component is preferably 20 to 500 $\mu$m, and more preferably 40 to 300 $\mu$m. When the thickness of the bottom surface component is within the above-described range, a suitable culture plate can be prepared without warpage of the bottom surface of the culture plate during culture of cells, etc.

The shape of the bottom surface component is not particularly limited as long as it can form at least one well when bonded to the well lateral wall component, and is preferably in the form of a plate, film or sheet.

The shape of the inside of the well of the bottom surface component include such as a flat bottom (F bottom), a round bottom (U bottom), a conical bottom (V bottom) and a flat bottom with a curved edge. In the case of processing into, for example, a round bottom (U bottom), a conical bottom (V bottom), or a flat bottom with a curved edge, it is also possible to produce the culture plate by producing a plate, a film or a sheet, and then performing secondary processing such as vacuum molding or air pressure molding. The shape of the inside of the well of the bottom surface component is selected according to a purpose of culture, but in two-dimensional culture of cells, etc., a flat bottom (F bottom) is typically preferable, and in three-dimensional culture, a round bottom (U bottom) or a conical bottom (V bottom) is typically preferable.

The method for producing the bottom surface component is not particularly limited, and equipment used for the production is also not limited. A plate, a film, a sheet or other molded article that forms the bottom surface component can be obtained by directly molding a material that constitutes the bottom surface component by a method such as extrusion molding, solution casting molding, injection molding or blow molding.

Specifically, for example, a normal inflation method or a T-die extrusion method is adopted as a method for forming the film or sheet. Typically, heating is applied during the production. In the case where a T-die extrusion method is

adopted, the extrusion temperature differs depending on the type of a material constituting the bottom surface component, and is preferably 100°C to 400°C, and particularly preferably 150°C to 300°C. The roll temperature is preferably 20°C to 100°C, and particularly preferably 30°C to 90°C.

The film or sheet may also be produced by a solution casting method in which a material constituting the bottom surface component is dissolved in a solvent, the solution is spread over resin or metal, and slowly dried while being leveled, thereby forming a film (forming a sheet). The solvent used is not particularly limited, and a hydrocarbon solvent such as cyclohexane, hexane, decane or toluene may be used. Two or more types of solvents may be mixed in consideration of the solubility and the drying efficiency of the material. The polymer solution can be applied by a method such as table coating, spin coating, dip coating, die coating, spray coating, bar coating, roll coating or curtain flow coating, dried, and peeled off to perform processing into a film or a sheet.

The bottom surface component, in particular, at least the culture surface of the bottom surface component, is preferably surface-treated. Here, the "culture surface" refer to the surface of the inside the well of the bottom surface component, which is, or is to be, in contact with cells, etc. when culturing cells, etc. using a well plate.

When surface treatment is performed on at least the culture surface of the bottom surface component, the culture surface can be brought into a state in which attachment of cells, etc. easily occurs, and cells, etc. easily proliferate uniformly. In addition, the wettability of the culture surface is enhanced, and at least one coating agent selected from a natural polymeric material, a synthetic polymeric material and an inorganic material is easily coated onto the culture surface. The coating will be described later.

The surface treatment is preferably performed before the bottom surface component and the well lateral wall component are bonded.

Examples of the surface treatment include hydrophilization treatment. The method used for the hydrophilization treatment is not particularly limited, and examples thereof include corona treatment, plasma treatment, ozone treatment, ultraviolet treatment, chemical vapor deposition, etching, or addition of a specific functional group such as a hydroxyl group, an amino group, a sulfone group, a thiol group or a carboxyl group, treatment with a specific functional group such as silane coupling, and surface roughening with such as an oxidant. Among them, surface hydrophilization treatment such as ultraviolet treatment, corona treatment, plasma treatment or ozone treatment is preferably performed in order to improve the wettability of the bottom surface component and enable efficient culturing of cells, etc. These surface treatments may be performed singly, or two or more thereof may be performed in combination.

Among these surface treatments, it is preferable that at least the culture surface of the bottom surface component be a plasma-treated culture surface because the surface can be brought into a state in which attachment easily occurs. Examples of the plasma treatment include a vacuum plasma treatment, a normal-pressure plasma treatment, and a highpressure plasma treatment. In the case where plasma treatment is performed, as a gas to be entrained, nitrogen, hydrogen, helium, oxygen or argon, for example, is used, and preferably at least one gas selected from nitrogen, helium and argon is selected.

The culture surface of the bottom surface component is preferably coated with at least one coating agent selected from a natural polymeric material, a synthetic polymeric material and an inorganic material.

The coating agent is not particularly limited. Examples of the natural polymeric material include collagen, gelatin, alginic acid, hyaluronic acid, glycosaminoglycans such as chondroitin sulfate, fibronectin, laminin, fibrinogen, osteopontin, tenascin, vitronectin, thrombospondin, agarose, elastin, keratin, chitosan, fibrin, fibroin, and saccharides. Examples of the synthetic polymeric material include polyglycolic acid, polylactic acid, polyethylene glycol, polycaprolactone, synthetic peptides, synthetic proteins, polyhydroxyethyl methacrylate, and polyethyleneimine. Examples of the inorganic material include β-tricalcium phosphate and calcium carbonate.

In addition, examples of the coating agent include vitrigel obtained by vitrifying hydrogel such as a conventional extracellular matrix component, and then rehydrating it. Mention is also made of, for example, collagen vitrigel which is formed of high-density collagen fiber networks produced from collagen, that is one of extracellular matrix components.

From the viewpoint of, for example, improving the ability to attach or proliferate cells, etc. and maintaining functions of cells, etc. for a longer period, the coating agent is preferably a protein such as collagen, gelatin, laminin or polylysine, or peptide, more preferably laminin, collagen or polylysine, and further more preferably collagen. One of these coating agents may be performed singly, or two or more thereof may be performed in combination.

It is preferable that the above-described coating treatment be performed on the bottom surface component because after the coating treatment, the well plate is not deformed, discolored or delaminated during washing with physiological saline or in a culture environment for cells, etc., and can be used as a culture plate while maintaining a stable initial state.

The water contact angle of the culture surface of the bottom surface component is preferably 30° or more, more preferably 35° or more, further more preferably 40° or more, particularly preferably 45° or more, and further more preferably 50° or more. The water contact angle is preferably 120° or less, more preferably 110° or less, further more preferably 100° or less, and particularly preferably 84° or less, or 70° or less. The upper limit and the lower limit can be arbitrarily combined.

When the water contact angle of the bottom surface component is adjusted to be within the above-described range, for example, cells, etc. easily attach to the bottom surface component, and cells, etc. easily proliferate uniformly on the bottom surface component. In addition, a natural polymeric material, a synthetic polymeric material or an inorganic material is easily coated uniformly onto the bottom surface component, and brought into close contact with the bottom surface component, and after the coating, the natural polymeric material, synthetic polymeric material or inorganic material does not peel during washing with physiological saline and in a cell culture environment, and can be used in cell culture while maintaining a stable initial state.
The water contact angle is measured by a static drop method. Details of the measurement method will be described later.

<Well lateral wall component>

The well lateral wall component is a component that constitutes the lateral wall of the well, and forms at least one well by bonding to the bottom surface component.
The surface free energy of the well lateral wall component is 18.0 to 34.0 mN/m. From the viewpoint of the drug adsorption property and processability, the surface free energy of the well lateral wall component is preferably 20.0 mN/m or more, more preferably 22.0 mN/m or more, and preferably 30.0 mN/m or less, more preferably 26.0 mN/m or less, and further more preferably 24.3 mN/m or less. These upper limit values and lower limit values can be arbitrarily combined.
If the surface free energy of the well lateral wall component is less than 18.0 mN/m, sufficient adhesive strength with the bottom surface component cannot be obtained. If the surface free energy of the well lateral wall component is more than 34.0 mN/m, a sufficient drug adsorption property cannot be obtained.
The well lateral wall component preferably has a glass transition temperature (Tg) measured by solid viscoelasticity temperature dispersion measurement of -10°C or higher, more preferably 30°C or higher, and further more preferably 120°C or higher. The upper limit of Tg is not particularly limited, and may be, for example, 300°C or lower.
When the Tg of the well lateral wall component is within the above-described range, excellent flexibility and strength are achieved. In addition, the molecules of the well lateral wall component hardly move even in a temperature environment of about 25 to 40°C, which is a typical temperature during culture of cells, etc., and as a result, a drug is hardly absorbed, which is preferable.
The material constituting the well lateral wall component is not particularly limited as long as the surface free energy is within the above-described range, and the material differs from that for the bottom surface component. For example, a known thermosetting resin or thermoplastic resin can be used.
The well lateral wall component preferably contains a thermoplastic resin among the materials mentioned above. The thermoplastic resin is not particularly limited, and examples thereof include polyolefin-based resins such as polyethylene, polypropylene, a cyclic olefin-based polymer, a polymethylpentene resin and a norbornene-based polymer; polymethacryl-based resins such as a polymethyl methacrylate resin; polyacryl-based resins such as a polymethyl acrylate resin; polyvinyl acetal resins; polyvinyl butyral resins; polyvinyl formal resins; polycarbonate resins; polyether ether ketone resins; polyether-based resins such as a polyether ketone resin; polyester-based resins; polyamide-based resins such as nylon-6, nylon-66, and polymetaxylene adipamide; polyamide imide resins; polyimide resins; polyether imide resins; styrene-based elastomers; polyolefin-based elastomers; polyurethane-based elastomers; polyester-based elastomers; polyamide-based elastomers; polytetrafluoroethylene resins; ethylene tetrafluoroethylene copolymers; polyvinylidene fluoride resins; polyvinyl fluoride resins; thermoplastic polyimide resins; polyvinylidene chloride resins; polyvinyl chloride resins; polyvinyl acetate resins; polyphenylene-based resins such as a polyphenylene oxide resin and a polyphenylene sulfide resin; polysulfone resins; polylactic acid resins; polyethersulfone resins; polyacrylonitrile resins, and styrene-acrylonitrile copolymer resins.
The thermoplastic resin is preferably a polyolefin-based resin, and the polyolefin-based resin is preferably at least one selected from a cyclic olefin-based copolymer ($\alpha$), a 4-methyl-1-pentene polymer ($\beta$), a propylene-based polymer ($\gamma$) and a cyclic olefin-based polymer ($\delta$) (except for the cyclic olefin-based copolymer ($\alpha$)).

(Cyclic olefin-based copolymer ($\alpha$))

Hereinafter, the cyclic olefin-based copolymer ($\alpha$) will be described, but the cyclic olefin-based copolymer ($\alpha$) is not limited to the following aspect.
The cyclic olefin-based copolymer ($\alpha$) is a copolymer of an $\alpha$-olefin (including ethylene) and a cyclic olefin.
The cyclic olefin compound constituting the cyclic olefin-based copolymer ($\alpha$) is not particularly limited, and examples thereof can include cyclic olefin monomers described in paragraphs 0037 to 0063 of WO2006/118261.
From the viewpoint that the heat resistance of the resulting component can be further improved while good transparency of the component is maintained, and moldability can be improved, the cyclic olefin-based copolymer ($\alpha$) preferably has a constituent unit (a) derived from at least one olefin represented by the following formula (I) and a

constituent unit (b) derived from at least one cyclic olefin selected from the group consisting of a constituent unit represented by the following formula (II), a constituent unit represented by the following formula (III), and a constituent unit represented by the following formula (IV).

[Chem. 1]

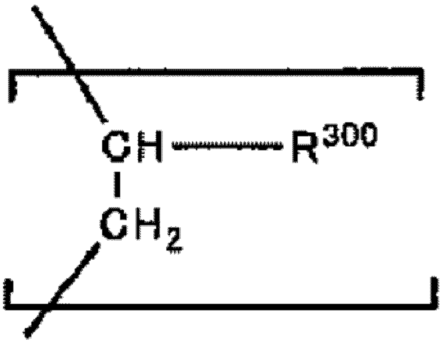

(I)

In the above formula (I), $R^{300}$ represents a hydrogen atom, or a linear or branched hydrocarbon group having 1 to 29 carbon atoms.

[Chem. 2]

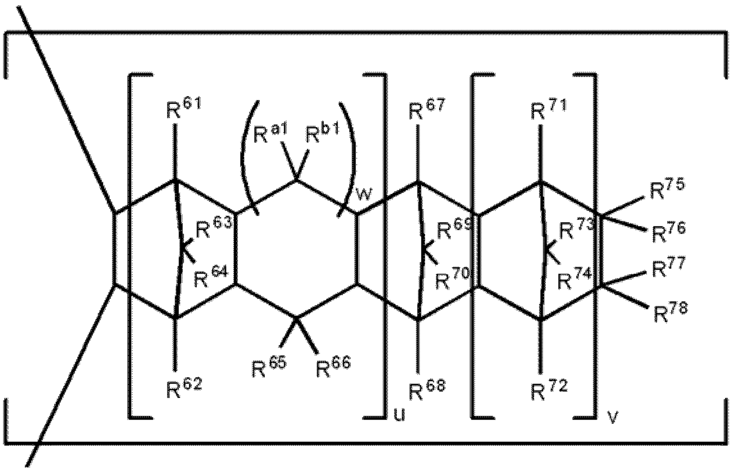

(II)

In the above (II), u is 0 or 1, v is 0 or a positive integer, preferably an integer of 0 or more and 2 or less, and more preferably 0 or 1, w is 0 or 1, $R^{61}$ to $R^{78}$ as well as $R^{a1}$ and $R^{b1}$ may be the same or different from each other, and are a hydrogen atom, a halogen atom, an alkyl group having 1 to 20 carbon atoms, a halogenated alkyl group having 1 to 20 carbon atoms, a cycloalkyl group having 3 to 15 carbon atoms, or an aromatic hydrocarbon group having 6 to 20 carbon atoms, and $R^{75}$ to $R^{78}$ may be bonded to each other to form a monocyclic ring or a polycyclic ring.

[Chem. 3]

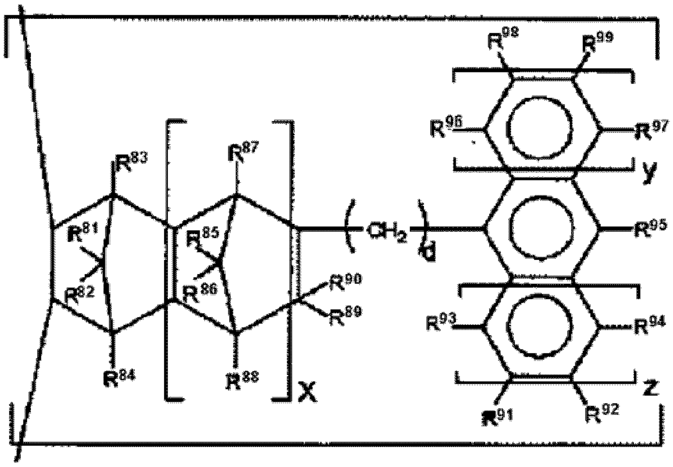

(III)

In the above formula (III), x and d are an integer of 0 or 1 or more, preferably an integer of 0 or more and 2 or less, and more preferably 0 or 1, y and z are 0, 1 or 2, $R^{81}$ to $R^{99}$ may be the same or different from each other, and are a hydrogen atom, a halogen atom, an aliphatic hydrocarbon group which is an alkyl group having 1 to 20 carbon atoms or a cycloalkyl group having 3 to 15 carbon atoms, an aromatic hydrocarbon group or an alkoxy group having 6 to 20 carbon atoms, a carbon atom to which $R^{89}$ and $R^{90}$ are bonded may be bonded directly or via an alkylene group having 1 to 3 carbon atoms to a carbon atom to which $R^{93}$ is bonded or a carbon atom to which $R^{91}$ is bonded, and $R^{95}$ and $R^{92}$ or $R^{95}$ and $R^{99}$ may be bonded to each other to form a monocyclic or polycyclic aromatic ring when y = z = 0.

[Chem. 4]

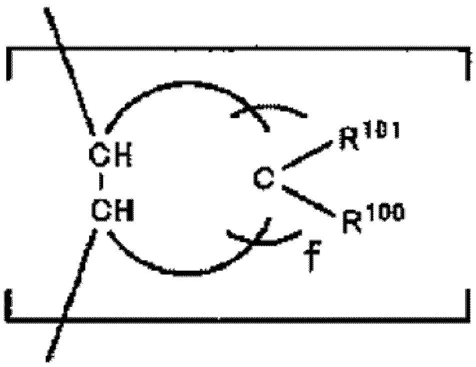

(IV)

In the above formula (IV), $R^{100}$ and $R^{101}$ may be the same or different from each other, and represent a hydrogen atom, or a hydrocarbon group having 1 to 5 carbon atoms, and f is $1 \leq f \leq 18$.

The olefin monomer which is one of copolymerization raw materials for the cyclic olefin-based copolymer ($\alpha$) undergoes addition copolymerization to form a constituent unit represented by the above formula (I). Specifically, an olefin monomer represented by the following formula (Ia) corresponding to the above formula (I) is used.

[Chem. 5]

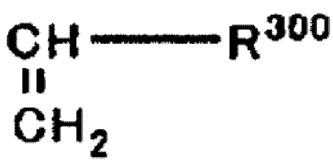

・・・ (I a)

In the above formula (Ia), $R^{300}$ represents a hydrogen atom, or a linear or branched hydrocarbon group having 1 to 29 carbon atoms.

Examples of the olefin monomer represented by the above formula (Ia) include ethylene, propylene, 1-butene, 1-pentene, 1-hexene, 3-methyl-1-butene, 3-methyl-1-pentene, 3-ethyl-1-pentene, 4-methyl-1-pentene, 4-methyl-1-hexene, 4,4-dimethyl-1-hexene, 4,4-dimethyl-1-pentene, 4-ethyl-1-hexene, 3-ethyl-1-hexene, 1-octene, 1-decene, 1-dodecene, 1-tetradecene, 1-hexadecene, 1-octadecene, and 1-eicosene. Among these, ethylene and propylene are preferable, and ethylene is particularly preferable, from the viewpoint of obtaining a well plate having further excellent heat resistance, mechanical characteristics and optical characteristics. Two or more types of olefin monomers represented by the above formula (Ia) may be used.

When the total of constituent units constituting the cyclic olefin-based copolymer ($\alpha$) is assumed to be 100% by mol, the proportion of the constituent unit (a) derived from the olefin is preferably 5% by mol or more and 95% by mol or less, more preferably 20% by mol or more and 90% by mol or less, further more preferably 40% by mol or more and 80% by mol or less, and particularly preferably 50% by mol or more and 70% by mol or less.

The proportion of the constituent unit (a) derived from the olefin can be measured by $^{13}$C-NMR.

The cyclic olefin monomer (b) which is one of copolymerization raw materials for the cyclic olefin-based copolymer ($\alpha$) undergoes addition copolymerization to form a constituent unit (b) derived from the cyclic olefin represented by the above formula (II), the above formula (III) or the above formula (IV). Specifically, cyclic olefin monomers (b) represented by formulae (IIa), (IIIa) and (IVa) corresponding to the above formulae (II), (III) and (IV), respectively, are used.

[Chem. 6]

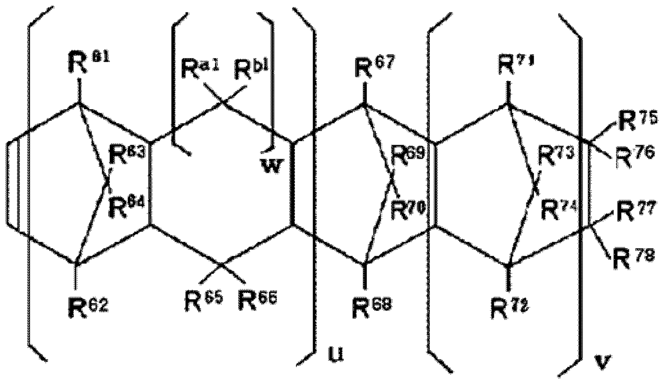

(IIa)

In the above formula (IIa), u is 0 or 1, v is 0 or a positive integer, preferably an integer of 0 or more and 2 or less, and more preferably 0 or 1, w is 0 or 1, $R^{61}$ to $R^{78}$ as well as $R^{a1}$ and $R^{b1}$ may be the same or different from each other, and are a hydrogen atom, a halogen atom, an alkyl group having 1 to 20 carbon atoms, a halogenated alkyl group having 1 to 20 carbon atoms, a cycloalkyl group having 3 to 15 carbon atoms, or an aromatic hydrocarbon group having 6 to 20 carbon atoms, and $R^{75}$ to $R^{78}$ may be bonded to each other to form a monocyclic ring or a polycyclic ring.

[Chem. 7]

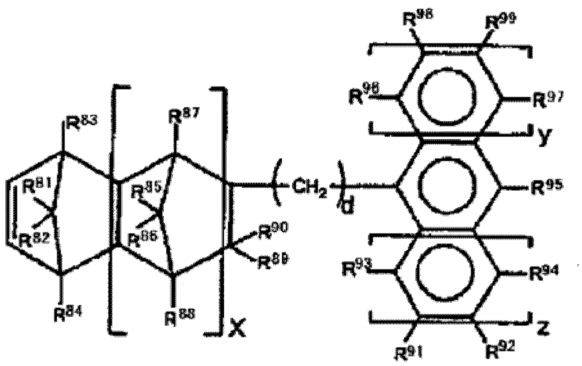

(IIIa)

In the above formula (IIIa), x and d are an integer of 0 or 1 or more, preferably an integer of 0 or more and 2 or less, and more preferably 0 or 1, y and z are 0, 1 or 2, $R^{81}$ to $R^{99}$ may be the same or different from each other, and are a hydrogen atom, a halogen atom, an aliphatic hydrocarbon group which is an alkyl group having 1 to 20 carbon atoms or a cycloalkyl group

having 3 to 15 carbon atoms, an aromatic hydrocarbon group or an alkoxy group having 6 to 20 carbon atoms, a carbon atom to which $R^{89}$ and $R^{90}$ are bonded may be bonded directly or via an alkylene group having 1 to 3 carbon atoms to a carbon atom to which $R^{93}$ is bonded or a carbon atom to which $R^{91}$ is bonded, and $R^{95}$ and $R^{92}$ or $R^{95}$ and $R^{99}$ may bonded to each other to form a monocyclic or polycyclic aromatic ring when y = z = 0.

[Chem. 8]

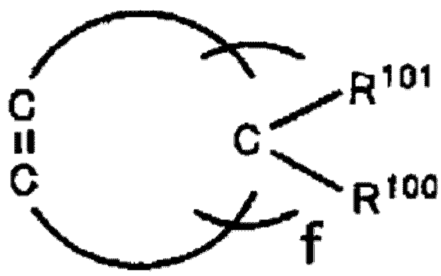

(IVa)

In the above formula (IVa), $R^{100}$ and $R^{101}$ may be the same or different from each other, and represent a hydrogen atom, or a hydrocarbon group having 1 to 5 carbon atoms, and f is $1 \leq f \leq 18$.

When the olefin monomer represented by the above formula (Ia) or the cyclic olefin monomer (b) represented by the formula (IIa), (IIIa) or (IVa) is used as a copolymerization component, the solubility of the cyclic olefin-based copolymer (α) in a solvent is further improved, so that moldability is improved, leading to improvement of the yield of the product.

As specific examples of the cyclic olefin monomer (b) represented by the formula (IIa), (IIIa) or (IVa), compounds described in paragraphs 0037 to 0063 of WO2006/118261 can be used.

Specific examples thereof include bicyclo-2-heptene derivatives (bicyclohept-2-ene derivatives), tricyclo-3-decene derivatives, tricyclo-3-undecene derivatives, tetracyclo-3-dodecene derivatives, pentacyclo-4-pentadecene derivatives, pentacyclopentadecadiene derivatives, pentacyclo-3-pentadecene derivatives, pentacyclo-4-hexadecene derivatives, pentacyclo-3-hexadecene derivatives, hexacyclo-4-heptadecene derivatives, heptacyclo-5-eicosene derivatives, heptacyclo-4-eicosene derivatives, heptacyclo-5-heneicosene derivatives, octacyclo-5-docosene derivatives, nonacyclo-5-pentacosene derivatives, nonacyclo-6-hexacosene derivatives, cyclopentadiene-acenaphthylene adducts, 1,4-methano-1,4,4a,9a-tetrahydrofluorene derivatives, 1,4-methano-1,4,4a,5,10,10a-hexahydro anthracene derivatives, and cycloalkylene derivatives having 3 to 20 carbon atoms.

Among the cyclic olefin monomers (b) represented by the formula (IIa), (IIIa) or (IVa), the cyclic olefin represented by the formula (IIa) is preferable.

As the cyclic olefin monomer (b) represented by the above formula (IIa), bicyclo[2.2.1]-2-heptene (also called norbornene), or tetracyclo $[6.2.1.1^{3,6}.0^{2,7}]$ dodec-4-ene (also called tetracyclo $[4.4.0.1^{2,5}.1^{7,10}]$-3-dodecene or tetracyclododecene) is preferably used, and tetracyclo$[6.2.1.1^{3,6}.0^{2,7}]$dodec-4-ene is more preferably used. These cyclic olefins have a rigid ring structure, and therefore give an advantage that the elastic moduli of the copolymer and the resulting component are easily maintained.

When the total of constituent units constituting the cyclic olefin-based copolymer (α) is assumed to be 100% by mol, the proportion of the constituent unit (b) derived from the cyclic olefin monomer (b) is preferably 5% by mol or more and 95% by mol or less, more preferably 10% by mol or more and 80% by mol or less, further more preferably 20% by mol or more and 60% by mol or less, and particularly preferably 30% by mol or more and 50% by mol or less.

The copolymer type of the cyclic olefin-based copolymer (α) is not particularly limited, and examples thereof can include a random copolymer or a block copolymer. In the present embodiment, a random copolymer is preferably used as the cyclic olefin-based copolymer (α) because of excellent transparency.

As the cyclic olefin-based copolymer (α), a random copolymer of ethylene and tetracyclo[4.4.0.12,5.17,10]-3-dodecene and a random copolymer of ethylene and bicyclo[2.2.1]-2-heptene are preferable, and a random copolymer of ethylene and tetracyclo[4.4.0.12,5.17,10]-3-dodecene is more preferable.

One of the cyclic olefin-based copolymers (α) may be used singly, or two or more thereof may be used in combination.

The cyclic olefin-based copolymer (α) can be produced by appropriately selecting conditions in accordance with the method of, for example, JP60-168708A, JP61-120816A, JP61-115912A, JP61-115916A, JP61-271308A, JP61-272216A, JP62-252406A, and JP62-252407A.

A commercially available cyclic olefin-based copolymer (α) may be used, and as the commercially available cyclic olefin-based copolymer (α), for example, APEL series (for example, APEL 6015T) manufactured by Mitsui Chemicals, Inc., Coxec series (for example, Coxec TCS-1) manufactured by KURABO INDUSTRIES LTD., and TOPAS series manufactured by Polyplastics Co., Ltd. can be used.

(4-Methyl-1-pentene polymer (β))

Hereinafter, the 4-methyl-1-pentene polymer (β) will be described, but the 4-methyl-1-pentene polymer (β) is not limited to the following aspect.

The 4-methyl-1-pentene polymer (β) includes homopolymers of 4-methyl-1-pentene, and copolymers of 4-methyl-1-pentene and other monomers. Hereinafter, the homopolymer of 4-methyl-1-pentene is also referred to as a 4-methyl-1-pentene homopolymer (β1).

The copolymer of 4-methyl-1-pentene and other monomers may be any of a random copolymer, an alternate copolymer, a block copolymer and a graft copolymer. As the copolymer of 4-methyl-1-pentene and other monomers, copolymers (β2) of 4-methyl-1-pentene and at least one olefin selected from ethylene and an α-olefin having 3 to 20 carbon atoms (except for 4-methyl-1-pentene) are preferable because the resulting component has high strength (is unlikely to break and crack), and hardly warps.

The 4-methyl-1-pentene polymer (β) is preferably at least one polymer selected from a 4-methyl-1-pentene homopolymer (β1), and a copolymer (β2) of 4-methyl-1-pentene and at least one olefin selected from ethylene and an α-olefin having 3 to 20 carbon atoms (except for 4-methyl-1-pentene), and more preferably a copolymer (β2) of 4-methyl-1-pentene and at least one olefin selected from ethylene and an α-olefin having 3 to 20 carbon atoms (except for 4-methyl-1-pentene).

Examples of the olefin include ethylene, propylene, 1-butene, 1-hexene, 1-heptene, 1-octene, 1-decene, 1-tetradecene, 1-hexadecene, 1-heptadecene, 1-octadecene, and 1-eicosene. The olefin can be appropriately selected according to physical properties necessary for a culture material. For example, from the viewpoint of moderate oxygen permeability and excellent rigidity, the olefin is preferably an α-olefin having 8 to 18 carbon atoms, and more preferably at least one selected from 1-octene, 1-decene, 1-dodecene, 1-tetradecene, 1-hexadecene, 1-heptadecene and 1-octadecene. When the number of carbon atoms in the olefin is in the above-described range, the film formation processability of the polymer is further improved, and as a result, deterioration in appearance due to cracking or breakage of end portions hardly occurs in release from a roll or mold during molding. Therefore, the rejection rate of the resulting components decreases.

One of the olefins may be contained singly, or two or more thereof may be combined. From the viewpoint of the strength of the material, the number of carbon atoms is preferably 10 or more. In the case where two or more different α-olefins are combined, it is particularly preferable to combine at least one selected from 1-tetradecene and 1-hexadecene and at least one selected from 1-heptadecene and 1-octadecene.

The content of constituent units derived from 4-methyl-1-pentene is preferably 60 to 100% by mol, and more preferably 80 to 98% by mol. The content of the constituent unit derived from at least one olefin selected from ethylene and α-olefins having 3 to 20 carbon atoms (except for 4-methyl-1-pentene) is preferably 0 to 40% by mol, and more preferably 2 to 20% by mol. Note that the content of the constituent units is based on the amount of all repeating constituent units, which is defined as 100% by mol. When the content of the constituent units is within the above-described range, a component that is excellent in processability and homogeneous is obtained, and warpage hardly occurs because of well-balanced toughness and strength.

The 4-methyl-1-pentene polymer (β) may have constituent units other than the constituent units derived from 4-methyl-1-pentene and the constituent units derived from the olefin (hereinafter, also referred to as "other constituent units") as long as the effects of the present invention are not impaired. The content of other constituent units is, for example, 0 to 10.0% by mol. In the case where the 4-methyl-1-pentene polymer (β) has other constituent units, there may be one type, or two or more types of other constituent units.

Examples of the monomer which derive other constituent units include cyclic olefins, aromatic vinyl compounds, conjugated dienes, non-conjugated polyenes, functional vinyl compounds, hydroxyl group-containing olefins, and halogenated olefins. As the cyclic olefins, aromatic vinyl compounds, conjugated dienes, non-conjugated polyenes, functional vinyl compounds, hydroxyl group-containing olefins, and halogenated olefins, for example, compounds described in paragraphs [0035]-[0041] of JP2013-169685A may be used.

One of the 4-methyl-1-pentene polymers (β) may be used singly, or two or more may be used in combination.

A commercially available 4-methyl-1-pentene (β) may be used, and as the commercially available 4-methyl-1-pentene polymer, TPX series manufactured by Mitsui Chemicals, Inc. can be used.

(Propylene-based polymer (γ))

Hereinafter, the propylene-based polymer (γ) will be described, but propylene-based polymer (γ) is not limited to the following aspect.

The propylene-based polymer (γ) is a polymer in which constituent units derived from propylene are contained in a proportion of more than 50% by mol and 100% by mol or less in the polymer.

Examples of the propylene-based polymer (γ) can include propylene homopolymers (γ1), or copolymers (γ2) of

propylene and at least one α-olefin having 2 to 20 carbon atoms excluding propylene. Here, examples of the α-olefin having 2 to 20 carbon atoms, other than propylene, include, ethylene, 1-butene, 1-pentene, 1-hexene, 4-methyl-1-pentene, 1-octene, 1-decene, 1-dodecene, 1-tetradecene, 1-hexadecene, 1-octadecene and 1-eicosene, and ethylene or an α-olefin having 4 to 10 carbon atoms is preferable.

The copolymer (γ2) of propylene and at least one α-olefin having 2 to 20 carbon atoms excluding propylene may be a random copolymer or a block copolymer. Constituent units derived from these α-olefins may be contained in a proportion of preferably 35% by mol or less, more preferably 30% by mol or less, in the copolymer (γ2) of propylene and at least one α-olefin having 2 to 20 carbon atoms excluding propylene.

The propylene-based polymer (γ) may be isotactic polypropylene, or syndiotactic polypropylene.

A commercially available propylene-based polymer (γ) may be used, and as the commercially available propylene-based polymer (γ), for example, NOVATEC BC03C manufactured by Japan Polypropylene Corporation and F107 manufactured by Prime Polymer Co., Ltd. can be used.

(Cyclic olefin-based polymer (δ))

Hereinafter, the cyclic olefin-based polymer (δ) will be described, but the cyclic olefin-based polymer (δ) is not limited to the following aspect.

The cyclic olefin-based polymer (δ) is a polymer which contains constituent units containing an alicyclic structure and which does not contain constituent units derived from at least one olefin represented by the above formula (I) (except for the cyclic olefin-based copolymer (α)). Examples of the alicyclic structure in the cyclic olefin-based polymer (δ) include saturated cyclic hydrocarbon (cycloalkane) structures, and unsaturated cyclic hydrocarbon (cycloalkene) structures. From the viewpoint of, for example, mechanical strength and heat resistance, cycloalkane structures and cycloalkene structures are preferable, and among them, cycloalkane structures are more preferable. The alicyclic structure may be present in the main chain, or in the side chain, and it is preferable that the alicyclic structure be contained in the main chain from the viewpoint of, for example, mechanical strength and heat resistance. There is no particular limitation on the number of carbon atoms constituting the alicyclic structure, and the characteristics of mechanical strength, heat resistance and moldability of the film are highly balanced when the number of the carbon atoms is in the range of typically 4 to 30, preferably 5 to 20, and more preferably 5 to 15.

The proportion of the constituent units containing an alicyclic structure, in the cyclic olefin-based polymer (δ) may be appropriately selected according to the intended use, and is preferably 50% by mass or more, more preferably 70% by mass or more, and particularly preferably 90% by mass or more. The proportion of the constituent units having an alicyclic structure, in the alicyclic structure-containing polymer is preferably in this range from the viewpoint of the transparency and heat resistance of the film.

Specific examples of the cyclic olefin-based polymer (δ) include (1) norbornene-based polymers, (2) monocyclic-ring cyclic olefin polymers, (3) cyclic conjugated diene-based polymers, (4) vinyl alicyclic hydrocarbon polymers, and hydrides of (1) to (4). Among these, hydrides of norbornene-based polymers, vinyl alicyclic hydrocarbon polymers and hydrides thereof are preferable from the viewpoint of, for example, heat resistance and mechanical strength.

(1) Norbornene-based polymer

Examples of the norbornene-based polymer include those obtained by ring-opening polymerization of a norbornene-based monomer.

Examples of the norbornene-based monomer include alicyclic compounds such as bicyclo[2.2.1]hept-2-ene (trivial name: norbornene), tricyclo[4.3.$0^{1,6}$.$1^{2,5}$]deca-3,7-diene (trivial name: dicyclopentadiene), 7,8-benzotricyclo [4.3.0.$1^{2,5}$]deca-3-ene (trivial name: methanotetrahydrofluorene, also referred to as 1,4-methano-1,4,4a,9a-tetrahydrofluorene) and tetracyclo[6.2.1.$1^{3,6}$.$0^{2,7}$]dodec-4-ene (trivial name: tetracyclododecene),as well as those having a substituent (for example, an alkyl group, an alkylene group, an alkylidene group or an alkoxycarbonyl group).

Examples of the polymer obtained by ring-opening polymerization include hydrogenated products of a ring-opened polymer of a norbornene-based monomer, such as ZEONEX (registered trademark; manufactured by Zeon Corporation), ZEONOR (registered trademark; manufactured by Zeon Corporation) or ARTON (registered trademark; manufactured by JSR Corporation).

Among these, hydrogenated products of a ring-opened polymer are preferable, and ring-opened polymers having no polar group (ZEONEX, ZEONOR) are particularly preferable, from the viewpoint of, for example, the high total light transmittance.

(2) Monocyclic-ring cyclic olefin-based polymer

As the monocyclic-ring cyclic olefin-based polymer, for example, addition polymers of a monocyclic-ring cyclic

olefin-based monomer such as cyclohexene, cycloheptene or cyclooctene can be used.

(3) Cyclic conjugated diene-based polymer

As the cyclic conjugated diene-based polymer, for example, polymers obtained by 1,2- or 1,4-addition polymerization of a cyclic conjugated diene-based monomer such as cyclopentadiene or cyclohexadiene, and hydrides thereof can be used.

(4) Vinyl alicyclic hydrocarbon polymer

Examples of the vinyl alicyclic hydrocarbon polymer include polymers of a vinyl alicyclic hydrocarbon-based monomer such as vinylcyclohexene or vinylcyclohexane, and hydrides thereof; and hydrides of an aromatic ring moiety of a polymer of a vinyl aromatic monomer such as styrene or α-methylstyrene, and the vinyl alicyclic hydrocarbon polymer may be any of, for example, copolymers such as random copolymers and block copolymers of a vinyl alicyclic hydrocarbon polymer or a vinyl aromatic monomer with other monomers that can be copolymerized with these monomers, and hydrides thereof. The block copolymer is not particularly limited, and examples thereof include diblock, triblock or higher multiblock and inclined-block copolymers.

(Silylated polyolefin (ε))

The well lateral wall component may further contain a silylated polyolefin (ε). It is preferable that the well lateral wall component contain a silylated polyolefin from the viewpoint of the drug adsorption property because the surface free energy of the well lateral wall component tends to decrease.

When the well lateral wall component contains a silylated polyolefin (ε), the well lateral wall component preferably contains a polyolefin-based resin, and more preferably contains a propylene-based polymer (γ) as the polyolefin-based resin.

Hereinafter, the silylated polyolefin (ε) will be described, but the silylated polyolefin (ε) is not limited to the following aspect.

The silylated polyolefin (ε) is a reaction product of a silicon-containing compound containing a constituent unit represented by the following formula (1) and a vinyl group-containing compound having a number-average molecular weight (Mn) of 100 or more and 500,000 or less as measured by gel permeation chromatography (GPC) (except for a case where the silicon-containing compound has 2 or more SiH groups per molecule and the vinyl group-containing compound has 2.0 or more vinyl groups on average per molecule, that is, a silylated polyolefin which is a reaction product of a silicon-containing compound having 2 or more SiH groups per molecule as the silicon-containing compound and a vinyl group-containing compound having a 2.0 or more vinyl groups on average per molecule as the vinyl group-containing compound, and a derivative thereof are used).

$$-Si(R^1)H-Y^1-\cdots \qquad (1)$$

In the above formula (1), $R^1$ is a hydrogen atom, a halogen atom or a hydrocarbon group, and $Y^1$ is O, S or $NR^{30}$ ($R^{30}$ is a hydrogen atom or a hydrocarbon group).

<Silicon-containing compound>

The silicon-containing compound constituting the silylated polyolefin (ε) is a hydrosilane compound having a constituent unit represented by the following formula (1).

$$-Si(R^1)H-Y^1\cdots \qquad (1)$$

In the above formula (1), $R^1$ is a hydrogen atom, a halogen atom or a hydrocarbon group, and $Y^1$ is O, S or $NR^{30}$ ($R^{30}$ is a hydrogen atom or a hydrocarbon group).

Examples of the halogen atom include fluorine, chlorine, bromine, and iodine.

The hydrocarbon group is preferably a hydrocarbon group having 2 to 40 carbon atoms, and more preferably a hydrocarbon group having 2 to 20 carbon atoms. Specific examples thereof include an alkyl group, an alkenyl group, and an aryl group. In $R^1$ and $R^{30}$, the hydrocarbon groups may be the same or different from each other.

Examples of the alkyl group include linear or branched alkyl groups such as a methyl group, an ethyl group, a n-propyl group, an isopropyl group, a n-butyl group, an isobutyl group, a tert-butyl group, a hexyl group, a 2-ethylhexyl group, an octyl group, a decyl group and an octadecyl group; cycloalkyl groups such as a cyclopentyl group, a cyclohexyl group

and a norbornyl group; and arylalkyl groups such as a benzyl group, a phenylethyl group and a phenylpropyl group.

Examples of the alkenyl group include a vinyl group, a propenyl group, and a cyclohexenyl group.

Examples of the aryl group include a phenyl group, a tolyl group, a dimethylphenyl group, a trimethylphenyl group, an ethylphenyl group, a propylphenyl group, a biphenyl group, a naphthyl group, a methylnaphthyl group, an anthryl group, and a phenanthryl group.

The above-described hydrocarbon group may contain one or more hetero atoms. Specific examples thereof include groups in which at least one hydrogen of these groups is replaced with a group containing a halogen atom, oxygen, nitrogen, silicon, phosphorus or sulfur.

In an embodiment, the silicon-containing compound has a structure represented by the following formula (2).

$$R^{22}\text{-}(Si(R^{21})H\text{-}Y^{21})_m\text{-}Z\text{-}(Y^{22}\text{-}Si(R^{23})H)_n\text{-}R^{24}\cdots \qquad (2)$$

In the above formula (2), $R^{21}$ and $R^{23}$ are each independently a hydrogen atom, a halogen atom, or a hydrocarbon group,

$R^{22}$ and $R^{24}$ are each independently a halogen atom, or a hydrocarbon group,
$Y^{21}$ and $Y^{22}$ are each independently O, S or $NR^{30}$ ($R^{30}$ is a hydrogen atom or a hydrocarbon group),
m is 0 or 1,
n is 0 or 1,
when a plurality of $R^{21}$, $R^{23}$, $Y^{21}$ and $Y^{22}$ are present, each group may be the same or different, and
Z is a divalent group represented by the following formula (3):

$$\text{-}Si(R^{41})(R^{41})\text{-}(Y^{23}\text{-}Si(R^{41})(R^{41}))_l\text{-}\cdots \qquad (3)$$

In the above formula (3), $R^{41}$ is a hydrogen atom, a halogen atom or a hydrocarbon group, each $R^{41}$ may be the same or different, $Y^{23}$ are each independently O, S or $NR^{30}$ ($R^{30}$ is a hydrogen atom or a hydrocarbon group), and l is an integer of 0 to 10,000.

When m = n = 0 in the above formula (2), at least one $R^{41}$ is a hydrogen atom in the above formula (3).

The halogen atom and the hydrocarbon group in the above formula (2) and the above formula (3) are as defined in the above formula (1).

In a typical embodiment, hydrocarbon groups in the above formulae (1), (2) and (3) are composed only of carbon atoms and hydrogen atoms.

In an embodiment, the silicon-containing compound has preferably 3 or more, more preferably 5 or more, and further more preferably 10 or more silicon atoms. The silicon-containing compound has preferably 10,000 or less, more preferably 1,000 or less, particularly preferably 300 or less, and further more preferably 50 or less silicon atoms.

In an embodiment, l in the above formula (3) is an integer of 0 to 10,000, and examples of the preferred upper limit and lower limit can include values determined by the values of m and n in the above formula (2) and the above preferred number of silicon atoms.

In an embodiment, a silicon-containing compound which meets m = n = 1 in the above formula (2), in other words, which has a SiH group at both ends is preferably used.

In an embodiment, a silicon-containing compound which meets m = 1 and n = 0 in the above formula (2), in other words, which has a SiH group at one end is preferably used.

Examples of the particularly preferred silicon-containing compound include compounds which meet m = n = 1 and in which all of $R^{21}$, $R^{23}$ and $R^{41}$ are hydrocarbon groups, in the above formulae (2) and (3). Each of the hydrocarbon groups may be the same or different.

Examples of another particularly preferred silicon-containing compound include compounds which meet m = 1 and n = 0 and in which $R^{21}$ and $R^{41}$ are all hydrocarbon groups, in the above formulae (2) and (3). Each of the hydrocarbon groups may be the same or different.

Specific examples of the silicon-containing compound used in the present embodiment are shown below. Examples of the silicon-containing compound according to the present embodiment include compounds having one SiH group.

Examples of the silicon-containing compound having one SiH group include compounds represented by the following formula (2a), and compounds in which a part or all of methyl groups have been substituted with a hydrocarbon group such as an ethyl group, a propyl group, a phenyl group or a trifloropropyl group, which may contain one or more hetero atoms, in the formula (2a).

$$HSi(CH_3)_2O\text{-}(\text{-}Si(CH^3)_2\text{-}O\text{-})_d\text{-}Si(CH_3)_3\cdots \qquad (2a)$$

(In the above formula (2a), d is an integer of 1 or more, where the upper limit is, for example, 1,000, preferably 300 and further more preferably 50.)

More specific examples of such compounds include, but are not limited to, compounds shown below.

$$C_4H_9\text{-}((CH_3)_2SiO)_9\text{-}(CH_3)_2SiH$$

$$C_4H_9\text{-}((CH_3)_2SiO)_{65}\text{-}(CH_3)_2SiH$$

Another examples of the silicon-containing compound having one SiH group include dimethylsiloxane/methylhydrogensiloxane copolymers represented by the following formula (2b), and compounds in which a part or all of methyl groups have been substituted with a hydrocarbon group such as an ethyl group, a propyl group, a phenyl group or a trifloropropyl group, which may contain one or more hetero atoms, in the following formula (2b).

$$Si(CH_3)_3O\text{-}(\text{-}Si(CH_3)_2\text{-}O\text{-})_e\text{-}(\text{-}SiH(CH_3)\text{-}O\text{-})\text{-}Si(CH_3)_3\cdots \qquad (2b)$$

(In the above formula (2b), e is an integer of 0 or more, where the upper limit is, for example, 1,000, preferably 300 and further more preferably 50.)

The order in which the $Si(CH_3)_2$-O- unit and the-$SiH(CH_3)$-O- unit are arranged is not particularly limited, and may be block, unregulated, or statistically random.

More specific examples of such compounds include, but are not limited to, compounds shown below.

$$Si(CH_3)_3O\text{-}SiH(CH_3)\text{-}O\text{-}Si(CH_3)_3$$

Examples of the silicon-containing compound according to the present embodiment also include compounds having two or more SiH groups.

Examples of the silicon-containing compound having two or more SiH groups include methylhydrogenpolysiloxane represented by the following formula (2c), and compounds in which a part or all of methyl groups have been substituted with a hydrocarbon group such as an ethyl group, a propyl group, a phenyl group or a trifloropropyl group, which may contain one or more hetero atoms, in the following formula (2c) .

$$(CH_3)_3SiO\text{-}(\text{-}SiH(CH_3)\text{-}O\text{-})_f\text{-}Si(CH_3)_3\cdots \qquad (2c)$$

(In the above formula (2c), f is an integer of 2 or more, where the upper limit is, for example, 1,000, preferably 300 and further more preferably 50.)

Another examples of the silicon-containing compound having two or more SiH groups include dimethylsiloxane/methylhydrogensiloxane copolymers represented by the following formula (2d), and compounds in which a part or all of methyl groups have been substituted with a hydrocarbon group such as an ethyl group, a propyl group, a phenyl group or a trifloropropyl group, which may contain one or more hetero atoms, in the following formula (2d).

$$(CH_3)_3SiO\text{-}(\text{-}Si(CH_3)_2\text{-}O\text{-})_g\text{-}(\text{-}SiH(CH_3)\text{-}O\text{-})_h\text{-}Si(CH_3)_3\cdots \qquad (2d)$$

(In the above formula (2d), g is an integer of 1 or more and h is an integer of 2 or more, where the upper limit of the total of g and h is, for example, 1,000, preferably 300 and further more preferably 50.)

In the above formula (2d), the order in which the $Si(CH_3)_2$-O- unit and the -$SiH(CH_3)$-O- unit are arranged is not particularly limited, and may be block, unregulated, or statistically random.

More specific examples of such compounds include, but are not limited to, compounds shown below.

[Chem. 9]

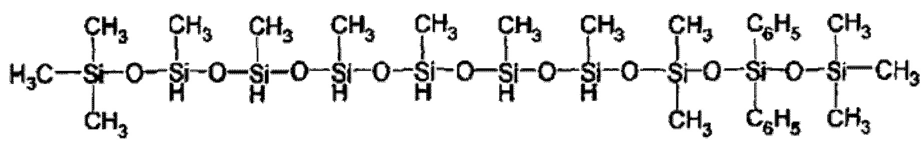

Still another examples of the silicon-containing compound having two or more SiH groups include methylhydrogenpolysiloxane represented by the following formula (2e), and compounds in which a part or all of methyl groups have been substituted with a hydrocarbon group such as an ethyl group, a propyl group, a phenyl group or a trifloropropyl group, which may contain one or more hetero atoms, in the following formula (2e).

$$HSi(CH_3)_2O\text{-}(\text{-}Si(CH_3)_2\text{-}O\text{-})_i\text{-}Si(CH_3)_2H\cdots \quad (2e)$$

(In the above formula (2e), i is an integer of 1 or more, where the upper limit is, for example, 1,000, preferably 300 and further more preferably 50.)

More specific examples of such compounds include, but are not limited to, compounds shown below.

$$HSi(CH_3)_2O\text{-}(\text{-}Si(CH_3)_2\text{-}O\text{-})_5\text{-}Si(CH_3)_2H$$

$$HSi(CH_3)_2O\text{-}(\text{-}Si(CH_3)_2\text{-}O\text{-})_8\text{-}Si(CH_3)_2H$$

$$HSi\ (CH_3)_2O\text{-}(\text{-}Si(CH_3)_2\text{-}O\text{-})_{18}\text{-}Si(CH_3)_2H$$

$$HSi\ (CH_3)_2O\text{-}(\text{-}Si(CH_3)_2\text{-}O\text{-})_{80}\text{-}Si(CH_3)_2H$$

$$HSi\ (CH_3)_2O\text{-}(\text{-}Si(CH_3)_2\text{-}O\text{-})_{230}\text{-}Si(CH_3)_2H$$

Still another examples of the silicon-containing compound having two or more SiH groups include methylhydrogenpolysiloxane represented by the following formula (2f), and compounds in which a part or all of methyl groups have been substituted with a hydrocarbon group such as an ethyl group, a propyl group, a phenyl group or a trifloropropyl group, which may contain one or more hetero atoms, in the following formula (2f).

$$HSi(CH_3)_2O\text{-}(\text{-}SiH(CH_3)\text{-}O\text{-})_j\text{-}Si(CH_3)_2H\cdots \quad (2f)$$

(In the above formula (2f), j is an integer of 1 or more, where the upper limit is, for example, 1,000, preferably 300 and further more preferably 50.)

Still another examples of the silicon-containing compound having two or more SiH groups include dimethylsiloxane/methylhydrogensiloxane copolymers represented by the following formula (2g), and compounds in which a part or all of methyl groups have been substituted with a hydrocarbon group such as an ethyl group, a propyl group, a phenyl group or a trifloropropyl group, which may contain one or more hetero atoms, in the following formula (2g).

$$HSi(CH_3)_2O\text{-}(\text{-}Si(CH_3)_2\text{-}O\text{-})_k\text{-}(\text{-}SiH(CH_3)\text{-}O\text{-})_l\text{-}Si(CH_3)_2H\cdots \quad (2g)$$

(In the above formula (2g), each of k and l is an integer of 1 or more, where the upper limit of the total of k and l is, for example, 1,000, preferably 300 and further more preferably 50.)

The order in which the $Si(CH_3)_2$-O- unit and the-$SiH(CH_3)$-O- unit are arranged is not particularly limited, and may be block, unregulated, or statistically random.

The number-average molecular weight (Mn) of the vinyl group-containing compound as measured by gel permeation chromatography (GPC) is typically 100 or more and 500,000 or less, and more preferably 100 or more and 100,000 or less. When the number-average molecular weight is equal to or greater than the above lower limit value, bleeding of the resulting silylated polyolefin from the material constituting the well lateral wall component can be further suppressed. When the number-average molecular weight is equal to or lower than the above upper limit value, the dispersibility of the silylated polyolefin in the material constituting the well lateral wall component is improved, so that variations in surface free energy of the plate can be reduced. In the present embodiment, the number-average molecular weight (Mn), the weight-average molecular weight (Mw) and Mw/Mn were values in terms of polyethylene as described later.

<Vinyl group-containing compound>

The vinyl group-containing compound constituting the silylated polyolefin (ε) is typically obtained by polymerization or copolymerization of one or more olefins selected from olefins having 2 to 50 carbon atoms.

Specific examples of the olefin having 2 to 50 carbon atoms include α-olefins such ethylene, propylene, 1-butene, 1-pentene, 3-methyl-1-butene, 1-hexene, 4-methyl-1-pentene, 3-methyl-1-pentene, 3,4-dimethyl-1-pentene, 4-methyl-1-hexene, 3-ethyl-1-pentene, 3-ethyl-4-methyl-1-pentene, 3,4-dimethyl-1-hexene, 4-methyl-1-heptene, 3,4-dimethyl-1-heptene, 1-octene, 1-decene, 1-dodecene, 1-tetradecene, 1-hexadecene, 1-octadecene, 1-eicosene and

vinylcyclohexane; olefins having an internal double bond, such as cis-2-butene and trans-2-butene; vinylidene compounds such as isobutene, 2-methyl-1-pentene, 2,4-dimethyl-1-pentene, 2,4-dimethyl-1-hexene, 2,4,4-trimethyl-1-pentene, 2,4-dimethyl-1-heptene, 2-methyl-1-butene, 2-methyl-1-hexene, 2-methyl-1-heptene, 2-methyl-1-octene, 2,3-dimethyl-1-butene, 2,3-dimethyl-1-pentene, 2,3-dimethyl-1-hexene, 2,3-dimethyl-1-octene, 2,3,3-trimethyl-1-butene, 2,3,3-trimethyl-1-pentene, 2,3,3-trimethyl-1-hexene, 2,3,3-trimethyl-1-octene, 2,3,4-trimethyl-1-pentene, 2,3,4-trimethyl-1-hexene, 2,3,4-trimethyl-1-octene, 2,4,4-trimethyl-1-hexene, 2,4,4-trimethyl-1-octene, 2-methyl-3-cyclohexyl-1-propylene, vinylidenecyclopentane, vinylidenecyclohexane, vinylidenecyclooctane, 2-methylvinylidenecyclopentane, 3-methylvinylidenecyclopentane and 4-methylvinylidenecyclopentane; arylvinyl compounds such as styrene, o-methylstyrene, m-methylstyrene, p-methylstyrene, 2,4-dimethylstyrene, o-ethylstyrene, m-ethylstyrene and p-ethylstyrene; arylvinylidene compounds such as α-methylstyrene, α-ethylstyrene and 2-methyl-3-phenylpropylene; functional group-substituted vinylidene compounds such as methyl methacrylate, ethyl methacrylate, methacrylic acid-n-propyl, isopropyl methacrylate, methacrylic acid-n-butyl, isobutyl methacrylate, methacrylic acid-tert-butyl, 2-cyanopropylene, 2-aminopropylene, 2-hydroxymethylpropylene, 2-fluoropropylene and 2-chloropropylene; aliphatic cyclic olefins having an internal double bond, such as cyclobutene, cyclopentene, 1-methyl-1-cyclopentene, 3-methyl-1-cyclopentene, 2-methyl-1-cyclopentene, cyclohexene, 1-methyl-1-cyclohexene, 3-methyl-1-cyclohexene, 2-methyl-1-cyclohexene, cycloheptene, cyclooctene, norbornene, 5-methyl-2-norbornene, tetracyclododecene, 5,6-dihydrodicyclopentadiene, 3a,4,5,6,7,7a-hexahydro-1Hindene, tricyclo[6.2.1.02,7]undec-4-ene, cyclopentadiene and dicyclopentadiene; cyclic olefins containing an aromatic ring, such as cyclopent-2-enylbenzene, cyclopent-3-enylbenzene, cyclohex-2-enylbenzene, cyclohex-3-enylbenzene, indene, 1,2-dihydronaphthalene, 1,4-dihydronaphthalene and 1,4-methano-1,4,4a,9a-tetrahydrofluorene; and cyclic polyenes having two or more double bonds and chain polyenes having two or more double bonds, such as butadiene, isoprene, 4-methyl-1,3-pentadiene, 4-methyl-1,4-pentadiene, 1,3-pentadiene, 1,4-pentadiene, 1,5-hexadiene, 1,4-hexadiene, 1,3-hexadiene, 1,3-octadiene, 1,4-octadiene, 1,5-octadiene, 1,6-octadiene, 1,7-octadiene, ethylidenenorbornene, vinylnorbornene, dicyclopentadiene, 7-methyl-1,6-octadiene, 4-ethylidene-8-methyl-1,7-nonadiene and 5,9-dimethyl-1,4,8-decatriene.

The olefin having 2 to 50 carbon atoms may have a functional group containing an atom such as oxygen, nitrogen or sulfur. Examples thereof include unsaturated carboxylic acids such as acrylic acid, fumaric acid, itaconic acid and bicyclo[2.2.1]hepta-5-ene-2,3-dicarboxylic acid and metal salts, such as sodium salts, potassium salts, lithium salts, zinc salts, magnesium salts and calcium salts, of any of these unsaturated carboxylic acids; unsaturated carboxylic anhydrides such as maleic anhydride, itaconic anhydride and bicyclo[2.2.1]hept-5-ene-2,3-dicarboxylic anhydride; unsaturated carboxylic acid esters such as methyl acrylate, ethyl acrylate, acrylic acid-n-propyl, isopropyl acrylate, acrylic acid-n-butyl, isobutyl acrylate, acrylic acid-tert-butyl and acrylic acid-2-ethylhexyl; vinyl esters such as vinyl acetate, vinyl propionate, vinyl caproate, vinyl caprate, vinyl laurate, vinyl stearate and vinyl trifluoroacetate; unsaturated glycidyl esters such as glycidyl acrylate, glycidyl methacrylate and itaconic acid monoglycidyl esters; halogenated olefins such as vinyl chloride, vinyl fluoride and allyl fluoride; unsaturated cyano compounds such as acrylonitrile, 2-cyano-bicyclo[2.2.1]hept-5-ene; unsaturated ether compounds such as methyl vinyl ether and ethyl vinyl ether; unsaturated amides such as acrylamide, methacrylamide and N,N-dimethylacrylamide; functional group-containing styrene derivatives such as methoxystyrene, ethoxystyrene, vinylbenzoic acid, methyl vinylbenzoate, vinylbenzyl acetate, hydroxystyrene, o-chlorostyrene, p-chlorostyrene and divinylbenzene; and N-vinylpyrrolidone.

In a preferred embodiment, the vinyl group-containing compound has a structure represented by the following formula (4), and has a number-average molecular weight of 100 or more and 500,000 or less.

$$A\text{-}CH = CH_2 \cdots \qquad (4)$$

Here, in the above formula (4), A is a polymer chain containing constituent units derived from one or more α-olefins having 2 to 50 carbon atoms.

In the above formula (4), the A moiety of the vinyl group-containing compound is preferably an ethylene polymer chain, a propylene polymer chain, or a copolymer chain of two or more olefins selected from the group consisting of α-olefins having 2 to 50 carbon atoms. The above α-olefin is preferably an α-olefin having 2 to 20 carbon atoms.

In a preferred embodiment, A of the vinyl group-containing compound represented by the above formula (4) is a polymer chain composed only of an α-olefin having 2 to 50 carbon atoms. Further more preferably, A of the vinyl group-containing compound is a polymer chain composed only of an α-olefin having 2 to 20 carbon atoms. Further more preferably, A of the vinyl group-containing compound is an ethylene homopolymer chain, a propylene homopolymer chain, or an ethylene/C3-C20 α-olefin copolymer chain.

The vinyl group-containing compound represented by the above formula (4) is preferably an ethylene/α-olefin copolymer in which the amount of constituent units derived from ethylene is in the range of 81 to 100% by mol, and the amount of constituent units derived from an α-olefin having 3 to 20 carbon atoms is in the range of 0 to 19% by mol. The vinyl group-containing compound is more preferably an ethylene/α-olefin copolymer in which the amount of constituent units derived from ethylene is in the range of 90 to 100% by mol, and the amount of constituent units derived from an α-olefin

having 3 to 20 carbon atoms is in the range of 0 to 10% by mol. Note that the total of constituent units derived from ethylene and constituent units derived from an α-olefin having 3 to 20 carbon atoms is 100% by mol. In particular, the amount of constituent units derived from ethylene is preferably 100% by mol.

The molecular weight distribution (ratio of weight-average molecular weight and number-average molecular weight, Mw/Mn) of the vinyl group-containing compound represented by the above formula (4) is preferably in the range of 1.1 to 3.0 as measured by gel permeation chromatography (GPC).

The number-average molecular weight (Mn) of the vinyl group-containing compound represented by the above formula (4) is preferably in the range of 100 or more and 500,000 or less, more preferably 100 or more and 100,000 or less, further more preferably 500 or more and 50,000 or less, and even more preferably 700 or more and 10,000 or less.

The melting point of the vinyl group-containing compound represented by the above formula (4) is preferably 70°C or higher and 130°C or lower.

Further more preferably, the vinyl group of the vinyl group-containing compound represented by the above formula (4) is preferably present at an end of the main chain, and the vinyl group is more preferably present only at an end of the main chain.

When the vinyl group-containing compound represented by the above formula (4) contains a vinyl group only at an end of the main chain, the end unsaturation calculated by $^1$H-NMR is preferably 60% by mol or more and 100% by mol or less. In one of further more preferred aspects, the end unsaturation calculated by $^1$H-NMR is 80% by mol or more and 99.5% by mol or less, and more preferably 90% by mol or more and 99% by mol or less.

For the vinyl group-containing compound represented by the above formula (4), for example, examples described in JP2017-155159A, in particular, paragraphs [0117] to [0156] of the same publication, can be utilized.

The silylated polyolefin used in the present embodiment may be one produced by any method, and for example, examples described in JP2017-155159A, in particular, paragraphs [0159] to [0189] of the same publication, can be utilized.

The silylated polyolefin (ε) is presumed to have a structure represented by any of formulae (5) to (9), for example. Of course, the combination of a silicon-containing compound and a vinyl group-containing compound is in no way to be limited to these examples.

[Chem. 10]

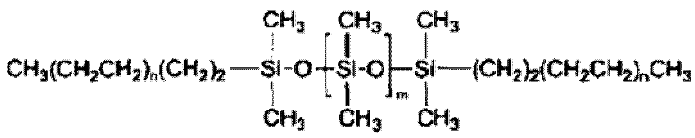

(5)

[Chem. 11]

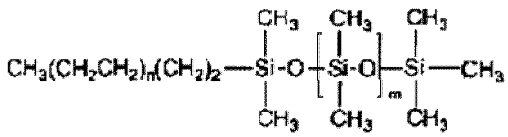

(6)

[Chem. 12]

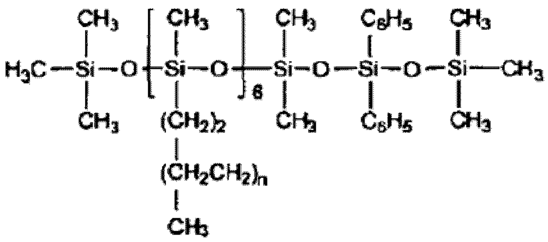

(7)

[Chem. 13]

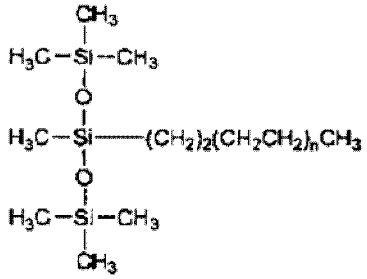

(8)

[Chem. 14]

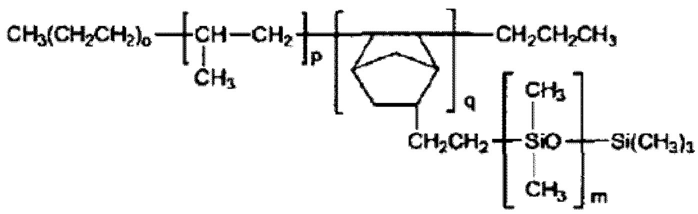

(9)

A commercially available silylated polyolefin (ε) may be used, and as the commercially available silylated polyolefin, Exfola series manufactured by Mitsui Chemicals, Inc. can be used.

The content of the silylated polyolefin (ε) in the material constituting the well lateral wall component is preferably 1 to 20% by mass, and more preferably 5 to 15% by mass, per 100% by mass of the material constituting the well lateral wall component.

(Glass)

For the bottom surface component, glass may be used, and examples thereof can include quartz glass, borosilicate glass, phosphate glass, and chemically strengthened glass. The glass constituting the bottom surface component may be of one type or two or more types.

The glass may be a commercially available product. As the commercially available glass, for example, NEO cover glass manufactured by Matsunami Glass Ind., Ltd. can be used.

In one of preferred aspects, the well lateral wall component of the well plate (X) contains the cyclic olefin-based copolymer (α) or the propylene-based polymer (γ) and the bottom surface component of the well plate (X) contains the 4-methyl-1-pentene polymer (β) from the viewpoint of the drug adsorption property. In one of preferred aspects, the well lateral wall component contains the 4-methyl-1-pentene polymer (β). In this case, it is more preferable that the bottom surface component contain the cyclic olefin-based copolymer (α), the propylene-based polymer (γ), the cyclic olefin-based polymer (δ) or borosilicate glass. In one of preferred aspects, the well lateral wall component of the well plate (X) contains the propylene-based polymer (γ) and silylated polyolefin (ε), and the bottom surface component of the well plate (X) contains the 4-methyl-1-pentene polymer (β).

<Method for culturing cell, tissue or organ>

An aspect of the present invention is a method for culturing a cell, tissue or organ, comprising the step of culturing a cell, tissue or organ using the well plate (X).

The method for culturing cells, etc. is not particularly limited, and may be performed according to a known protocol, and a commercially available culture medium or culture kit may be used.

Culturing is typically performed in a culture medium placed inside a well of the well plate (X). Culturing in a culture medium means that at least a part of cells, etc. is immersed in a culture medium and cultured, and whole of cells, etc. need not be immersed in the culture medium.

The culture medium is not particularly limited as long as it is a culture medium enabling cells, etc. to proliferate, and may be appropriately selected according to a type of cells, etc. to be used.

The amount of the culture medium used for culture and the frequency of replacement of the culture medium is not particularly limited. The culture temperature is not particularly limited, but is typically about 25 to 40°C.
The culture period is not particularly limited, and cells, etc. may be cultured until cells, etc. are sufficiently attached to the culture plate and a desired confluency is obtained.

## Examples

Hereinafter, the present disclosure will be described in further detail with reference to Examples and Comparative Examples, but the present disclosure is not limited thereto in any way.

[Production Example 1] Production of well plate frame (A-1)

A 24-well plate frame in which bottom surfaces of wells are opened were formed by injection molding using NOVATEC BC03C manufactured by Japan Polypropylene Corporation, as a propylene-based polymer (γ). The surface free energy of the formed well plate frame (A-1) was 32.2 mN/m. The method for measuring the surface free energy will be described later.

[Production Example 2] Production of well plate frame (A-2)

A 24-well plate frame was formed in the same manner as in Production Example 1 except that APEL 6015T manufactured by Mitsui Chemicals, Inc. was used as a cyclic olefin-based copolymer (α). The surface free energy of the formed well plate frame (A-2) was 29.0 mN/m.

[Production Example 3] Production of well plate frame (A-3)

A 24-well plate frame was formed in the same manner as in Production Example 1, except that a composition obtained by dry-blending 10 parts by mass of a silylated polyolefin, which had been obtained according to Synthesis Example 6 of JP5575929 as a silylated polyolefin (ε) with 90 parts by mass of Polypropylene F107 manufactured by Prime Polymer Co., Ltd. as a propylene-based polymer (γ) was used in place of the propylene-based polymer (γ). The surface free energy of the formed well plate frame (A-3) was 24.7 mN/m.

[Production Example 4] Production of well plate frame (A-4)

A 24-well plate frame was formed in the same manner as in Production Example 1, except that TPX manufactured by Mitsui Chemicals, Inc. (total light transmittance 92%, glass transition temperature 40°C, weight-average molecular weight (Mw) $42.8 \times 10^4$) was used as a 4-methyl-1-pentene polymer (β). The surface free energy of the formed well plate frame (A-4) was 24.0 mN/m. The methods for measuring the total light transmittance, the glass transition temperature and the weight-average molecular weight (Mw) will be described later.

[Production Example 5] Production of bottom surface components (B-1) and (B-2)

TPX manufactured by Mitsui Chemicals, Inc. (total light transmittance 93.7%, glass transition temperature 40°C, weight-average molecular weight (Mw) $42.8 \times 10^4$) was used as a 4-methyl-1-pentene polymer (β), and put in a T-die extruder equipped with a full-flight-type screw for extruding a base material layer. The extrusion temperature was set to 270°C, the roll temperature was set to 60°C, and extrusion molding was performed while changing the condition of the roll rotation speed, thereby obtaining two films different in thickness. A 50 μm-thick film was taken as a bottom surface component (B-1), and a 400 μm-thick film was taken as a bottom surface component (B-2).
The content of the 4-methyl-1-pentene polymer (β) was 99.5% by mass per 100% by mass of the bottom surface component (B-1) or (B-2). The content of constitutional units derived from 4-methyl-1-pentene when the amount of all repeating constituent units of the 4-methyl-1-pentene polymer (β) is assumed to be 100% by mol was 98.4% by mol in both the components.

[Production Example 6] Production of bottom surface component (B-5)

Pellets of a norbornene-based polymer (a hydrogenated product of a ring-opened polymer of a norbornene-based monomer, corresponding to a cyclic olefin-based polymer (δ), ZEON OR1420, manufactured by ZEON CORPORATION; glass transition temperature Tg: 136°C) were dried at 100°C for 4 hours using a hot-air dryer through which air was circulated. The pellets were extruded at 260°C using a single screw extruder with a polymer filter (filtration accuracy 30

μm) and a T-shaped die, and the extruded sheet-shaped norbornene-based polymer was cooled through a cooling drum to obtain a 50 μm-thick cyclic olefin-based polymer (δ) film, which was taken as a bottom surface component (B-5).

[Production Example 7] Production of culture plate

A 100 μm-thick polypropylene film (corresponding to the propylene-based polymer (γ), CTP1147 manufactured by TOYOBO Co., Ltd.) was taken as a bottom surface component (B-3).
A 25 μm-thick cyclic olefin-based copolymer film (corresponding to the cyclic olefin-based copolymer (α), Coxec TCS-1 manufactured by KURABO INDUSTRIES LTD.) was taken as a bottom surface component (B-4).
NEO cover glass (manufactured by Matsunami Glass Ind., Ltd., product code C024601) was taken as a bottom surface component (B-6).
In a chamber filled with a nitrogen flow, plasma treatment (treatment rate: 2 m/min, output 4.5 kW, two reciprocations) was performed on the bottom surface components (B-1) to (B-6) using a normal-pressure plasma surface treatment apparatus (manufactured by Sekisui Chemical Company, Limited).
The plasma-treated bottom surface components (B-1) to (B-6) were brought into close contact with the bottom surfaces of the well plate frames (A-1) to (A-4) with a medical adhesive (manufactured by 3M Company) interposed therebetween, thereby obtaining well plates. The obtained well plate was packaged in a gamma ray-resistant bag and sterilized by irradiation with a gamma ray of 10 kGy. Then a 0.5 mg/mL collagen solution was added to each the well of the well plate at 0.5 mL/well, and an excess collagen solution was then removed. This was allowed to stand at room temperature for 30 to 60 minutes, then washed with Dulbecco PBS (-), and dried overnight at room temperature to produce culture plates 1 to 8. Table 1 below shows combinations of the well plate frames (A-1) to (A-4) and the bottom surface components (B-1) to (B-6) used for the culture plates 1 to 8. The culture plates 1 to 8 were taken as Examples 1 to 8, respectively.

(Evaluation of cell attachment)

To the culture plates 1 to 8, a cell suspension of frozen rat primary liver cells was added at 0.5 mL/well to seed the cells at a cell density of $1 \times 10^5$ cells/$cm^2$. The cells were incubated at 37°C under 5% $CO_2$, and states after culturing for 1 day and 7 days were observed under a microscope and evaluated according to the following criteria. Table 1 shows the results.

A: A state is observed in which liver cells are attached to the culture surface and spread out.
B: A state is observed in which liver cells are attached to the culture surface but rounded and not spread out, or partially peeled. Alternatively, liver cells are not attached to the culture surface.

(Evaluation of drug adsorption property)

To arbitrary three wells of the culture plates 1 to 8, an evaluation drug solution 1 was added at 0.5 mL per well. The cells were allowed to stand at 23°C for 2 days, and the drug solution was then collected. The concentration of the collected evaluation drug solution 1 was measured by LC/MS, and the ratio (%) to the concentration of the evaluation drug solution 1 before addition to the culture plates 1 to 8 was calculated. An average value for the three wells was determined, and taken as a drug residual ratio. Table 1 shows the results. The higher the drug residual ratio indicates a well plate with lower the drug adsorption property.
A drug residual ratio was similarly calculated except that the evaluation drug solution 1 was replaced with an evaluation drug solution 2, and three wells were arbitrarily selected from 21 wells which had not been used in the evaluation with the evaluation drug solution 1. Table 1 shows the results.
Evaluation drug solution 1: solution of ciclosporin A in dimethyl sulfoxide (hereinafter, referred to as DMSO) (concentration 10 μmol/L)
Evaluation drug solution 2: solution of isoproterenol hydrochloride in DMSO (concentration 10 μmol/L)

<LC/MS conditions>

- Apparatus: Acquity UPLC I-class system/TQ-S micro (water)
- Ionization method: electrospray ionization (ESI), detection of positive and negative ions

•Detection: selective reaction monitoring (SRM)

1. Polarity:
Positive: ciclosporin A, isoproterenol hydrochloride
2. Precursor ions:

Ciclosporin A: m/z 1203
Isoproterenol hydrochloride: m/z 212

3. Product ions:

Ciclosporin A: m/z 156
Isoproterenol hydrochloride: m/z 152

(Measurement of surface free energy)

Using a contact angle meter of model A-XE (manufactured by Kyowa Interface Science Co., Ltd.), the contact angle of the well plate frame (test material) was measured with water, diiodo-methane and 1-bromonaphthalene used as standard test liquids 1 to 3. By a calculation method based on the Kitazaki-Hata theory for calculation of surface free energy described later, the surface free energy of the test material was calculated using the known surface free energy of the standard test liquid and the measured value of the contact angle of the standard test liquid on the test material. For the calculation, multifunctional integration analysis software F AMAS (manufactured by Kyowa Interface Science Co., Ltd.) which is application software accompanying the contact angle meter was used. The numerical value shown in Table 1 is an average value of the results of contact angle measurement obtained by conducting the same test three times for one test material.

Hereinafter, the Kitazaki-Hata theory will be described in more detail.

When the surface free energy of the well plate frame to be calculated is A, and the components of the surface free energy A are a component ($A_d$) belonging to a dispersion force, a component ($A_p$) belonging to an orientation (polarity) force and a component ($A_h$) belonging to a hydrogen bonding force, A is given by equation (10). The intermolecular force coming from an induction force is very small, and therefore is ignored in calculation.
[Math. 1]

$$A = A_d + A_p + A_h \quad \cdot\cdot\cdot \text{ Equation (10)}$$

When the surface free energy of the standard test liquid 1 in which the values of each component are known is $B_1$, and the components of the surface free energy $B_1$ are a component ($B_{1d}$) belonging to a dispersion force, a component ($B_{1p}$) belonging to an orientation (polarity) force and a component ($B_{1h}$) belonging to a hydrogen bonding force, $B_1$ is given by equation (11).
[Math. 2]

$$B_1 = B_{1d} + B_{1p} + B_{1h} \quad \cdot\cdot\cdot \text{ Equation (11)}$$

Similarly, when the surface free energy of the standard test liquid 2 in which the values of each component are known is $B_2$, and the surface free energy of a liquid 3 in which the values of each component are known is $B_3$, $B_2$ and $B_3$ are given by equations (12) and (13), respectively.
[Math. 3]

$$B_2 = B_{2d} + B_{2p} + B_{2h} \quad \cdot\cdot\cdot \text{ Equation (12)}$$

[Math. 4]

$$B_3 = B_{3d} + B_{3p} + B_{3h} \quad \cdots \text{Equation (13)}$$

Further, when the contact angle measured using the well plate frame and the standard test liquid 1 is $\theta_1$, the contact angle between the well plate frame and the standard test liquid 2 is $\theta_2$, and the contact angle between the well plate frame and the standard test liquid 3 is $\theta_3$, the relationships between each component of the surface free energy of the well plate frame and the standard test liquid 1, the standard test liquid 2 and the standard test liquid 3, and the values of the contact angle satisfy the relationships shown in equation (14), equation (15) and equation (16).
[Math. 5]

$$\sqrt{A_d \cdot B_{1d}} + \sqrt{A_p \cdot B_{1p}} + \sqrt{A_h \cdot B_{1h}} = \frac{B_1(1+\cos\theta_1)}{2} \quad \text{Equation (14)}$$

[Math. 6]

$$\sqrt{A_d \cdot B_{2d}} + \sqrt{A_p \cdot B_{2p}} + \sqrt{A_h \cdot B_{2h}} = \frac{B_2(1+\cos\theta_2)}{2} \quad \text{Equation (15)}$$

[Math. 7]

$$\sqrt{A_d \cdot B_{3d}} + \sqrt{A_p \cdot B_{3p}} + \sqrt{A_h \cdot B_{3h}} = \frac{B_3(1+\cos\theta_3)}{2} \quad \text{Equation (16)}$$

Simultaneous linear equations with three unknowns consisting of the above equations (14), (15) and (16) is solved to calculate $A_d$, $A_p$ and $A_h$, and further the surface free energy A of the well plate frame is calculated by equation (10).

(Measurement of glass transition temperature of bottom surface component)

The glass transition temperature (Tg) of the bottom surface component of each of the culture plates 1 to 7 was measured with a differential scanning calorimeter according to Japanese Industrial Standard JIS-K7121: 1987 (Method for measuring transition temperatures of plastics).
The glass transition temperature (Tg) of borosilicate glass which is the bottom surface component of the culture plate 8 was measured with a differential thermal dilatometer (TMA) according to Japanese Industrial Standard JIS-R3103-3 (Method for measuring transition temperatures by thermal dilatation method).

(Measurement of glass transition temperature of well lateral wall component)

For the glass transition temperature (Tg) of the well lateral wall component of each of the culture plates 1 to 7, solid viscoelasticity temperature dispersion measurement was performed under the following conditions, and the peak top value of the obtained loss tangent (tan $\delta$) was taken as a glass transition temperature (Tg) of the well lateral wall component.

(Measurement Conditions)

Measurement apparatus: DVA-225 manufactured by IT Keisoku Seigyo K.K.
Deformation mode: tension
Temperature range: -30°C to 200°C
Temperature increase rate: 3°C/min

Frequency: 1 Hz
Strain: 0.05%
Atmospheric environment: air

(Measurement of total light transmittance)

For the measurement sample, the total light transmittance was measured in accordance with JIS K 7361-1 using a haze meter (NDH2000 manufactured by NIPPON DENSHOKU INDUSTRIES Co., Ltd.).

[Measurement of Water Contact Angle]

The water contact angle was measured according to Japanese Industrial Standard JIS-R3257 (Testing method for wettability of substrate glass surface). A water droplet with a volume of 4 μL or less, which can be considered to have a spherical shape under constant temperature and humidity conditions of 25 ± 5°C and 50 ± 10%, was dropped onto a surface of a measurement sample, and by a static drop method, the angle of a contact interface between the measurement sample and the water droplet within 1 minute immediately after the water droplet came into contact with the surface of the measurement sample was measured.

[Comparative Example 1]

A commercially available a 24-well TCPS (tissue culture polystyrene) culture plate having a bottom surface thickness of 1,000 μm (manufactured by Corning Incorporated, model number: NCO3524, the bottom surface component and the well plate frame are made of polystyrene with a hydrophilized surface) was used as a culture plate 9. Evaluation was performed in the same manner as in Examples 1 to 8 except that a different culture plate was used. Table 1 shows the results.

[Comparative Example 2]

A commercially available 24-well culture plate having a bottom surface component thickness of 350 μm (product name: G-Plate, manufactured by VECELL Co., Ltd., model number: V24WGPB-10, the bottom surface component is made of PDMS (polydimethylsiloxane) and the well plate frame is made of polystyrene with a hydrophilized surface) was used as a culture plate 10. Evaluation was performed in the same manner as in Examples 1 to 8 except that a different culture plate was used. Table 1 shows the results.

[Comparative Example 3]

In a chamber filled with a nitrogen flow, plasma treatment (treatment rate: 2 m/min, output 4.5 kW, two reciprocations) was performed on the bottom surface component (B-1) using a normal-pressure plasma surface treatment apparatus (manufactured by Sekisui Chemical Company, Limited). The bottom surface component of the culture plate 10 was peeled to provide a 24-well plate frame made of polystyrene with a hydrophilized surface in which the bottom surfaces of wells are opened (surface free energy is 43.0 mN/m), and the plasma-treated bottom surface component (B-1) was brought into close contact with the bottom surface with a medical adhesive (manufactured by 3M Company) interposed therebetween, thereby obtaining a well plate. The obtained well plate was packaged in a gamma ray-resistant bag and sterilized by irradiation with a gamma ray of 10 kGy. Then a 0.5 mg/mL collagen solution was added to each the well of the well plate at 0.5 mL/well, and an excess collagen solution was then removed. This was allowed to stand at room temperature for 30 to 60 minutes, then washed with Dulbecco PBS (-), and dried overnight at room temperature to obtain a well plate of Comparative Example 3 as a culture plate 11. The obtained well plate was evaluated in the same manner as in Examples 1 to 8 except that a different culture plate was used. Table 1 shows the results.

[Comparative Example 4]

Except that the bottom surface component (B-3) was used in place of the bottom surface component (B-1), the same process as in Comparative Example 3 was carried out to prepare a well plate, which was taken as a culture plate 12. Evaluation was performed in the same manner as in Examples 1 to 8 except that a different culture plate was used. Table 1 shows the results.

[Table 1]

| | | Example 1 | Example 2 | Example 3 | Example 4 | Example 5 | Example 6 | Example 7 | Example 8 | Comparative Example 1 | Comparative Example 2 | Comparative Example 3 | Comparative Example 4 |
|---|---|---|---|---|---|---|---|---|---|---|---|---|---|
| | Culture plate No. | 1 | 2 | 3 | 4 | 5 | 6 | 7 | 8 | 9 | 10 | 11 | 12 |
| Well lateral component | Well plate frame No. | A-1 | A-1 | A-2 | A-3 | A-4 | A-4 | A-4 | A-4 | PS | PS | PS | PS |
| | Material | Propylene-based polymer (γ) | Propylene-based polymer (γ) | Cyclic olefin-based copolymer (α) | Propylene-based polymer (γ)+ silylated polyolefin (ε) | 4-Methyl-1-pentene polymer (β) | 4-Methyl-1-pentene polymer (β) | 4-Methyl-1-pentene polymer (β) | 4-Methyl-1-pentene polymer (β) | Polystyrene with hydrophilized surface | Polystyrene with hydrophilized surface | Polystyrene with hydrophilized surface | Polystyrene with hydrophilized surface |
| | Surface free energy (mN/m) | 32.2 | 32.2 | 29.0 | 24.7 | 24.0 | 24.0 | 24.0 | 24.0 | 43.0 | 43.0 | 43.0 | 43.0 |
| | Tg(°C) | -5 | -6 | 145 | -8 | 40 | 40 | 40 | 40 | 94 | 94 | 94 | 94 |
| Bottom surface component | Bottom surface component No. | B-1 | B-2 | B-1 | B-1 | B-3 | B-4 | B-5 | B-6 | PS | PDMS | B-1 | B-3 |
| | Material | 4-Methyl-1-pentene polymer (β) | 4-Methyl-1-pentene polymer (β) | 4-Methyl-1-pentene polymer (β) | 4-Methyl-1-pentene polymer (β) | Propylene-based polymer (γ) | Cyclic olefin-based polymer (σ) | Cyclic olefin-based polymer (δ) | Borosilicate glass | Polystyrene with hydrophilized surface | Polydimethyl siloxane | 4-Methyl-1-pentene polymer (β) | Propylene-based polymer (γ) |
| | Thickness (μm) | 50 | 400 | 50 | 50 | 100 | 25 | 50 | 150 | 1000 | 350 | 50 | 100 |
| | Tg(°C) | 40 | 40 | 40 | 40 | -20 | 78 | 136 | 530 | 100 | -120 | 40 | -20 |
| | Total light transmittance (%) | 93.7 | 93.7 | 93.7 | 93.7 | 90.0 | 91.3 | 92.8 | 90.2 | 98.1 | 92.0 | 93.7 | 90.0 |
| | Water contact angle (°) | 51.5 | 50.4 | 53.3 | 53.3 | 62.2 | 57.5 | 58.4 | 50.1 | 60.6 | 76.6 | 57.5 | 60.8 |
| Cell attachment (culture for 1 day) | | A | A | A | A | A | A | A | A | A | A | A | A |

(continued)

| | Example 1 | Example 2 | Example 3 | Example 4 | Example 5 | Example 6 | Example 7 | Example 8 | Comparative Example 1 | Comparative Example 2 | Comparative Example 3 | Comparative Example 4 |
|---|---|---|---|---|---|---|---|---|---|---|---|---|
| Cell attachment (culture for 7 days) | A | A | A | A | A | A | A | A | A | B | A | A |
| Drug residual ratio (cyclosporine A) | 76% | 77% | 80% | 93% | 93% | 95% | 94% | 95% | 56% | 2% | 58% | 53% |
| Drug residual ratio (isoproterenol hydrochloride) | 78% | 76% | 83% | 90% | 91% | 96% | 94% | 96% | 65% | 1% | 63% | 55% |

The culture plates 1 to 8 were excellent in ability to attach cells, etc., and had a low drug adsorption property. In particular, the culture plate 4 in which the well lateral wall component contains a propylene-based polymer ($\gamma$) and a silylated polyolefin ($\varepsilon$) and the bottom surface component contains a 4-methyl-1-pentene polymer ($\beta$), the culture plate 5 in which the well lateral wall component contains a 4-methyl-1-pentene polymer ($\beta$) and the bottom surface component contains a propylene-based polymer ($\gamma$), the culture plate 6 in which the well lateral wall component contains a 4-methyl-1-pentene polymer ($\beta$) and the bottom surface component contains a cyclic olefin-based copolymer ($\alpha$), the culture plate 7 in which the well lateral wall component contains a 4-methyl-1-pentene polymer ($\beta$) and the bottom surface component contains a cyclic olefin-based polymer ($\delta$), and the culture plate 8 in which the well lateral wall component contains a 4-methyl-1-pentene polymer ($\beta$) and the bottom surface component contains borosilicate glass were excellent in ability to attach cells, etc., and had a very low drug adsorption property. On the other hand, the culture plates 9 to 12 were excellent in ability to attach cells, etc., but had a high drug adsorption property, and did not give satisfaction when used as a culture plate.

## Claims

1. A well plate comprising a bottom surface component and a well lateral wall component,

   wherein the bottom surface component and the well lateral wall component are bonded to each other to form at least one well,
   the bottom surface component and the well lateral wall component are formed of different materials, and
   a surface free energy of the well lateral wall component is 18.0 to 34.0 mN/m.

2. The well plate according to claim 1, wherein the well lateral wall component comprises a thermoplastic resin.

3. The well plate according to claim 2, wherein the thermoplastic resin is a polyolefin-based resin.

4. The well plate according to claim 3, wherein the polyolefin-based resin is at least one selected from a cyclic olefin-based copolymer ($\alpha$), a 4-methyl-1-pentene polymer ($\beta$), a propylene-based polymer ($\gamma$) and a cyclic olefin-based polymer ($\delta$) (except for the cyclic olefin-based copolymer ($\alpha$)).

5. The well plate according to claim 4, wherein the well lateral wall component further comprises a silylated polyolefin ($\varepsilon$).

6. The well plate according to claim 1 or 2, wherein a glass transition temperature of the bottom surface component is -40°C or higher as measured in accordance with JIS-K 7121: 1987.

7. The well plate according to claim 1 or 2, wherein a total light transmittance of the bottom surface component is 80.0% or more as measured in accordance with JIS-K-7361-1.

8. The well plate according to claim 1 or 2, wherein a thickness of the bottom surface component is 20 to 500 $\mu$m.

9. The well plate according to claim 1 or 2, wherein a culture surface of the bottom surface component is coated with at least one coating agent selected from a natural polymeric material, a synthetic polymeric material and an inorganic material.

10. The well plate according to claim 1 or 2, wherein a water contact angle of the culture surface of the bottom surface component is 50 to 100°.

11. The well plate according to claim 1 or 2, wherein the glass transition temperature of the well lateral wall component is 30°C or higher as measured by solid viscoelasticity temperature dispersion measurement.

12. The well plate according to claim 11, wherein the glass transition temperature of the well lateral wall component is 120°C or higher as measured by solid viscoelasticity temperature dispersion measurement.

13. The well plate according to claim 1 or 2, wherein the surface free energy of the well lateral wall component is 18.0 to 24.3 mN/m.

14. A method for culturing a cell, tissue or organ, comprising the step of culturing a cell, tissue or organ using the well plate

according to claim 1 or 2.

## INTERNATIONAL SEARCH REPORT

International application No.
**PCT/JP2023/019310**

**A. CLASSIFICATION OF SUBJECT MATTER**

***C12M 1/00***(2006.01)i; ***B32B 3/12***(2006.01)i; ***C12N 5/071***(2010.01)i
FI: C12M1/00 C; B32B3/12 Z; C12N5/071

According to International Patent Classification (IPC) or to both national classification and IPC

**B. FIELDS SEARCHED**

Minimum documentation searched (classification system followed by classification symbols)

C12N5/071; B32B3/12; C12M1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | JP 6-277037 A (KAWASUMI LAB INC) 04 October 1994 (1994-10-04)<br>claims, examples | 1-14 |
| Y | 竹澤　俊明, 細胞の足場となる新素材「コラーゲンビトリゲル膜」の開発と その医薬学分野での実用化構想, 薬剤学, 2015, vol. 75, no. 6 , pp. 344-353<br>fig. 6, (TAKEZAWA, Toshiaki. Development of a Novel Material “Collagen Vitrigel Membrane” Functioned as a Cellular Scaffold and Its Concept for the Practical Applications in Medical and Pharmaceutical Fields. Journal of Pharmaceutical Science and Technology, Japan.) | 1-14 |
| Y | JP 2016-67322 A (FUJIFILM CORP) 09 May 2016 (2016-05-09)<br>claims, examples | 1-14 |
| Y | WO 2020/256079 A1 (MITSUI CHEMICALS, INC.) 24 December 2020 (2020-12-24)<br>claims, paragraphs [0011], [0065], etc. | 1-14 |
| Y | JP 2021-50331 A (TOPPAN PRINTING CO LTD) 01 April 2021 (2021-04-01)<br>paragraph [0031] | 1-14 |

☐ Further documents are listed in the continuation of Box C.　☑ See patent family annex.

* Special categories of cited documents:
“A” document defining the general state of the art which is not considered to be of particular relevance
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **01 August 2023** | **08 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**
**Information on patent family members**

International application No.
**PCT/JP2023/019310**

| Patent document cited in search report | Publication date (day/month/year) | Patent family member(s) | Publication date (day/month/year) |
|---|---|---|---|
| JP 6-277037 A | 04 October 1994 | (Family: none) | |
| JP 2016-67322 A | 09 May 2016 | WO 2016/052078 A1<br>claims, examples | |
| WO 2020/256079 A1 | 24 December 2020 | US 2021/0309954 A1<br>claims, paragraphs [0019], [0099] | |
| JP 2021-50331 A | 01 April 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022064159 A
- WO 2006118261 A
- JP 60168708 A
- JP 61120816 A
- JP 61115912 A
- JP 61115916 A
- JP 61271308 A
- JP 61272216 A
- JP 62252406 A
- JP 62252407 A
- JP 2013169685 A
- JP 2017155159 A
- JP 5575929 B